# EUROPEAN PATENT APPLICATION

(11) **EP 1 489 098 A1**
(43) Date of publication of application: **22.12.2004**
(21) Application number: 02743822.5
(22) Date of filing: 04.07.2002
(51) Int. Cl.: C07K 16/18, C12N 5/20, C12P 21/08

(54) **PRODUCTION OF HYBRIDOMA PRODUCING ANTIHUMAN CENP-A PEPTIDE MONOCLONAL ANTIBODY AND METHOD OF USING THE SAME**

(30) Priority: 31.01.2002 JP 2002023587
(71) Applicant: Japan Science and Technology Agency, Kawaguchi-shi, Saitama 332-0012 (JP)
(72) Inventor: YODA, Kinya LM Hikarigaoka B1-16, Nagoya-shi Aichi 464-0006 (JP); NOZAKI, Naohito, Kanagawa 238-0023 (JP); OKAZAKI, Tsuneko, Aichi 466-0815 (JP)
(74) Representative: Cresswell, Thomas Anthony
(86) International application number: PCT/JP2002/006773
(87) International publication number: WO 2003/064474

(57) **Abstract**

It is intended to provide antibodies, in particular, monoclonal antibodies against, as antigens, peptides comprising N-terminal 1/3 amino acid sequences of a protein CENP-A expressed in centromeres in chromosome and a process for producing the same. Antibodies against partial-length peptides of the CENP-A protein expressed in centromeres in chromosome (preferably a peptide having the amino acid sequence of 3-to 19-positions of the CENP-A protein, a peptide having the amino acid sequence of 27-to 40-positions thereof or a peptide having the amino acid sequence of 126- to 140-positions thereof); and a process for producing the same. A method of solubilizing a centromere chromatin while sustaining its completeness by treating with 0.3 M NaCl and MNase. A method of using an anti-CENP-A antibody which comprises chemically bonding the antibody to a support and isolating and purifying the same by the immunoprecipitation method.

SEQUENCE LISTING

<110> JAPAN SCIENCE AND TECHNOLOGY AGENCY

<120> PRODUCTION OF HYBRIDOMA PRODUCING ANTIHUMAN CENP-A PEPTIDE
MONOCLONAL ANTIBODY AND METHOD OF USING THE SAME

<130> N.91930

<140> EP 02743822.5
<141> 2002-07-04

<150> PCT/JP02/06773
<151> 2002-07-04

<150> JP 2002-023587
<151> 2002-01-31

<160> 2

<170> PatentIn version 3.2

<210> 1
<211> 29
<212> DNA
<213> Artificial sequence

<220>
<223> Oligonucleotide primer

<400> 1
ggccggacca tggctgcgtc cggtctgga 29

<210> 2
<211> 29
<212> DNA
<213> Artificial sequence

<220>
<223> Oligonucleotide primer

<400> 2
atagaatcct cttcagctgg tttagccat 29

## Description

### Technical Field

The present invention relates to an antibody against a CENP-A protein, a method of producing the same and a method of using the same. More specifically, the present invention relates to a monoclonal antibody against, as an antigen, a peptide composed of 1/3 of the N-terminal amino acid sequence of a protein CENP-A expressed in the centromere in chromosome, a method of producing the same and a method of using the same. In addition, the present invention relates to a method of solubilizing a chromatin protein and a method of detecting, identifying and quantitating a chromatin protein using the same. The antibody of the present invention is useful in detection or identification of a chromosomal aberration, and also useful in elucidation of the structure or the mechanism of a centromere.

### Background Art

A centromere plays a key role in migrating to the pole by binding to a M-phase spindle of a eukaryotic cell that undergoes mitosis. During mitosis, protein complexes called kinetochores are formed on both ends of the centromere, and microtubules (kinetochore microtubules) which form spindles bind to these kinetochores. In vertebrate cells, the kinetochore, which is formed at the centromere at mitosis and is the attachment site for spindle microtubules, is a three-layered disc-shaped structure composed of a dense inner plate, a middle lucent domain and a dense outer plate.

Centromere proteins known to date include constitutive proteins, such as CENP-A, -B, -C and -H that are present at the centromere throughout the cell cycle, and transient proteins such as CENP-E, -F, INCENP, Mad1, Mad2, Bub1, Bub2 and RubR1 that appear after the onset of M-phase. The constitutive proteins appear as dots in nuclei at S-phase, which are called "pre-kinetochore", since at least one of them has been reported to be located in the inner kinetochore plate at M-phase.

A CENP-B gene codes for an 80 kDa protein that binds to a 17 bp DNA motif known as a CENP-B box, which is present in human α-satellite and mouse minor satellite DNA, and causes nucleosome positioning (to align at a certain position on the nucleotide sequences) around the CENP-B box region. The CENP-B gene seems to be non-essential, since knockout mice of this gene grow without any apparent defect in growth and morphology.

A CENP-C gene is essential for chromosome segregation and its gene product (a 140 kDa protein) is a DNA-binding protein without apparent sequence specificity. It has been reported that CENP-C is detected at the inner kinetochore plate by electron microscopy, while CENP-B is located in the pairing domain.

A CENP-A gene codes for a histone H3 variant. The C-terminal 2/3 of CENP-A is highly homologous to the histone H3, but the remaining 1/3 of the amino-terminus is unique to CENP-A. The domain composed of histone homologous sequences located in the C-terminal region is essential for targeting CENP-A to the centromeric region. A mouse CENP-A gene has been shown to be an essential gene by the gene targeting method. CSE4 that is a CENP-A homologue has been isolated from S. cerevisiae and has been also shown to be essential for chromosome segregation. By the point mutation analysis, the functional domains of Cse4p have been shown to be distributed across the histone-fold domain, which is necessary for interaction with histone H4, and the amino-terminal 33 peptides. A CENP-A homologue has been identified in C. elegans, D. melanogaster and S. pombe and is also essential for chromosome segregation.

In S. cerevisiae, regarding the centromere region, a 125 bp DNA sequence known as "CEN sequence" is genetically defined, and 8 or more proteins associated with a centromere including Cse4p are localized in this DNA region to form a higher-order chromatin complex which is resistant to nuclease attack. In S. pombe, a much longer DNA sequence (40 to 120 kb), composed of a 4 to 7 kb unique sequence (cc region) flanked by tens of kilobases of an inverted repeat sequence, forms the centromere region. It is speculated that some higher-order chromatin structure may be formed in the cc region, since in this region, nucleosome ladders by MNase cleavage become smear when an active centromere is formed. The centromeres of higher eukaryotes contain hundreds to thousands of kilobases of tandemly repeated DNA sequences, although their sequences and unit lengths totally vary depending on the species (52).

In humans, α-satellite (alphoid) sequences varying from 500 kb to 5 Mb are found at the centromere regions. The α-satellite sequence is, in each chromosome, composed of a higher-order sequence in which a 171 bp α-satellite repeat sequence further contains several types unique to respective chromosomes, which is further repeated to form a long sequence ranging over several megabases, and is composed of 171 bp monomer repeats that show chromosome-specific variation in a sequence and in a higher-order repeat arrangement. However, a stable neocentromere has been found in an euchromatic region lacking a repetitive DNA sequence such as α-satellite.

Epigenetical establishment of neocentromeres has been reported in human, D. melanogaster and S. pombe. This suggests that the formation of a DNA-protein complex, especially the formation of a CENP-A nucleosome, is critically important for the formation of an active centromere. A nucleosome containing CENP-A may be what distinguishes centromeric chromatin from euchromatin or heterochromatin, and promotes the formation of a functional kinetochore. The present inventors have previously shown that CENP-A can replace histone H3 in a nucleosomal reconstitution system in vitro, and that the basic structure of the CENP-A nucleosome is the same as that of a H3 nucleosome composed of an octamer of core histones (H3, H4, H2A and H2B) with nucleosomal DNA wrapped around it. Therefore, the present inventors propose that a centromeric chromatin complex composed of a CENP-A nucleosome and other centromere proteins should constitute a pre-kinetochore complex.

To further elucidate above proposal, a method such as chromatin immunoprecipitation method (CHIP) has been awaited in order to isolate a centromeric chromatin complex from HeLa cells, and in addition, to elucidate that a CENP-A nucleosome is selectively formed on α-satellite containing a CENP-B box, and CENP-B and -C bind to nucleosomal DNA to form a chromatin complex of A/B/C pre-kinetochore.

Incidentally, a method using an antibody is extremely useful in identifying or isolating a specific protein, and a method of producing an antibody using various mammals is widely applied. On the implementation of protein engineering (so called proteomics) in which a protein complex is isolated using an antibody and its constitutive factors are to be exhaustively analyzed, a demand for high specificity and quality uniformity of an antibody is getting increased, especially, a demand for a monoclonal antibody is getting increased.

A monoclonal antibody keeps proliferating and producing an antibody indefinitely once a hybridoma cell that produces an antibody is established by cloning, since the cell is an immortalized cell (tumor cell). Accordingly, it becomes possible to obtain infinitely the antibody with uniform specificity.

However, in a conventional method of producing a monoclonal antibody, selection and establishment of a hybridoma cell that produces a target antibody are extremely complicated. In particular, in the case where a synthesized relatively short peptide of an amino acid chain is used as an antigen, an epitope (site recognized by an antibody) can be clearly restricted, however, it is not known whether or not the monoclonal antibody can be actually used until it is produced by the complicated method. Therefore, a large number of relatively short peptides had to be prepared and the complicated work had to be conducted over a long period of time.

### Disclosure of the invention

The present invention provides an antibody, in particular, a monoclonal antibody against, as an antigen, a peptide composed of 1/3 of the N-terminal amino acid sequence of a protein CENP-A expressed in the centromere in chromosome, and a method of producing the same.

The present invention relates to an antibody against a partial-length peptide of a CENP-A protein expressed in the centromere in chromosome.

In addition, the present invention relates to, in a method of producing a monoclonal antibody by a cell fusion method, a method of producing a monoclonal antibody against a CENP-A protein characterized in that P3-X63-Ag8.653 cell, which is a mouse myeloma cell, is used as a myeloma cell, and GIT medium is used as a medium.

The present invention relates to a method of solubilizing centromeric chromatin, more specifically, a method of solubilizing centromeric chromatin by treatment with micrococcal nuclease (MNase). The solubilization of the present invention is characterized in that solubilization can be performed while retaining the integrity of chromatin.

Furthermore, the present invention relates to a method of detecting, identifying or quantitating a protein contained in centromeric chromatin by immunoprecipitating the solubilized centromeric chromatin proteins with the use of a CENP antibody, preferably a CENP-A antibody, preferably a monoclonal antibody. According to the present invention, it is revealed that a protein associated with differentiation as well as a protein associated with cell division are contained in chromatin and by analyzing the protein contained in centromeric chromatin, it is possible to diagnose various diseases such as a cancer.

### Brief Description of the Drawings

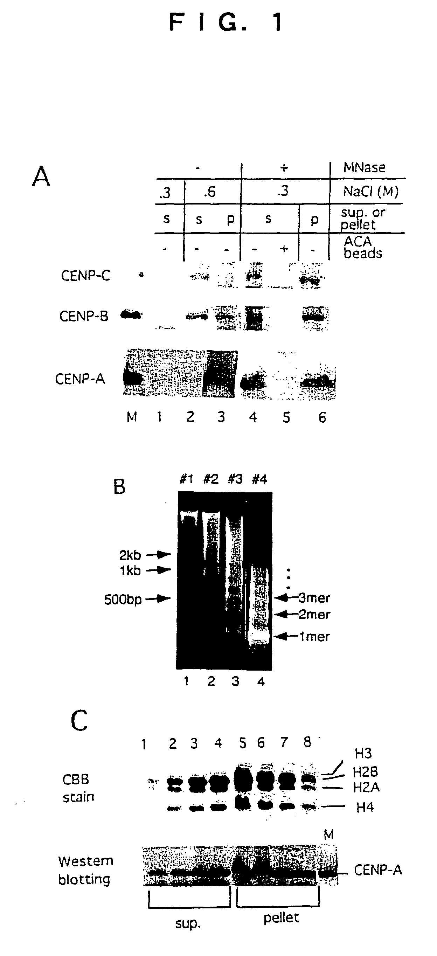

Figs. 1 are photographs substituted for drawings which show the solubilization of centromeric chromatin (Fig. 1A), the size distribution of DNA in the solubilized centromeric chromatin (Fig. 1B), the results of the staining of a soluble fraction and an insoluble fraction after MNase digestion of a bulk chromatin protein (Fig. 1 C. upper panel), and the results of the staining of CENP-A by Western blotting (Fig. 1C. lower panel).
Fig. 1A is a photograph substituted for a drawing which shows the results of examining the conditions for solubilizing centromeric chromatin from the nuclei of HeLa cells. Lane M of Fig. 1A shows the band positions of CENP-A, -B and -C. Lane 1 of Fig. 1A shows the result of the supernatant obtained by the treatment of the nuclei with 0.3 M NaCl. Lane 2 of Fig. 1A shows the result of the supernatant obtained by the treatment with 0.6 M NaCl, and Lane 3 of Fig. 1A shows the result of the pellet in this case. Lane 4 of Fig. 1A shows the result of the supernatant of the case where the nuclei were digested with micrococcal nuclease (MNase) in 0.3 M NaCl. Lane 5 of Fig. 1A shows the result of the case where ACA beads were added thereto. Lane 6 of Fig. 1A shows the result of the pellet of the case where the nuclei were digested with MNase in 0.3 M NaCl.
Fig. 1B is a photograph substituted for a drawing which shows the size distribution of the DNA in the chromatin after MNase digestion. Lane 1 shows the sample of 20 U/ml for 2 minutes (40 U/ml x min., sample #1); lane 2, 20 U/ml for 4 minutes (80 U/ml x min., sample #2); lane 3, 40 U/ml for 5 minutes (200 U/ml x min., sample #3); lane 4, 80 U/ml for 45 minutes (3,600 U/ml x min., sample #4).
Fig. 1C is a photograph substituted for a drawing which shows the results of detecting a soluble fraction and an insoluble fraction after digesting bulk chromatin proteins with MNase by fractionating with SDS-PAGE and by staining with Coomassie brilliant blue (CBB) (Fig. 1C. upper panel), and the results of identifying CENP-A by immunostaining with the use of an ACA antibody after transferring the fractionated proteins to a membrane (Fig. 1C. lower panel). Lanes 1 to 4 of Fig. 1C correspond to the samples from the soluble fraction, and lanes 5 to 8 correspond to the samples from the insoluble fraction. Lane M shows the position of CENP-A.

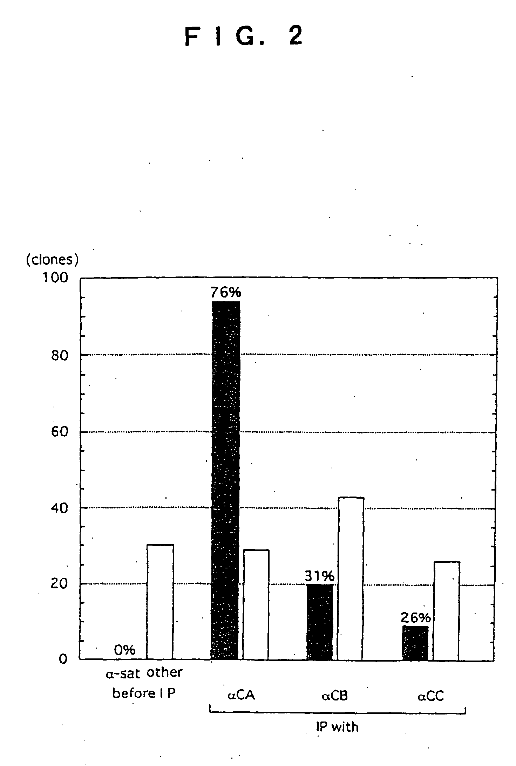

Fig. 2 shows the distribution of the DNA sequences obtained by the immunoprecipitation of the bulk chromatin solubilized by MNase digestion from HeLa nuclei with the anti-CENP-A, the anti-CENP-B, or the anti-CENP-C antibody of the present invention, the cloning of the DNA and the determination of the nucleotide sequences. The black bars of Fig. 2 show the number of clones with α-satellite DNA that contains a CENP-B box. The open bars show the number of clones other than these.

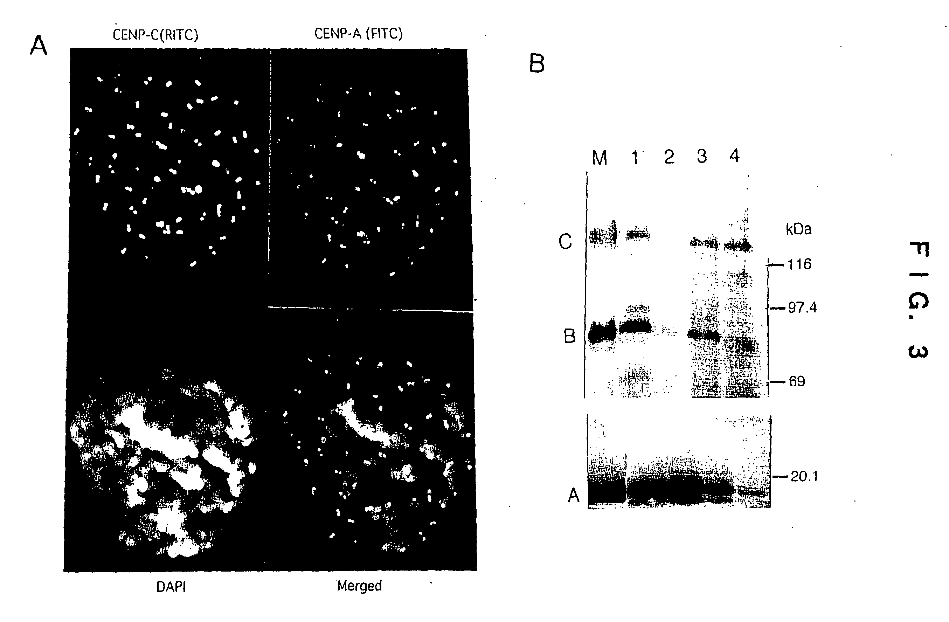

Fig. 3A is an immunofluorescence micrograph of mitotic chromosomes from HeLa cells, which is a photograph substituted for a drawing. The upper left of Fig. 3A is what was stained with an anti-CENP-C antibody and the upper right of Fig. 3A is what was stained with an anti-CENP-A antibody. For these staining procedures, anti-mouse IgG-fluorescein isothiocyanate (FITC) for the CENP-A antibody, and anti-guinea pig IgG-rhodamine isothiocyanate (RITC) for the CENP-C antibody were used as the secondary antibodies. The lower left of Fig. 3A is what was obtained by staining the chromosome with 4',6'-diamidino-3-phenylindole (DAPI). The lower right of Fig. 3A is what was obtained by merging the three panels.
Fig. 3B is a photograph substituted for a drawing which shows the results of detecting, by the Western blot method, CENP-A, CENP-B, and CENP-C which were coprecipitated with the anti-CENP-A antibody or CENP-C when performing immunoprecipitation (CHIP) of the solubilized chromatin by the anti-CENP-A antibody or the anti-CENP-C antibody of the present invention. The samples were obtained by treating the isolated nuclei of HeLa cells with 40 U/ml of MNase for 5 minutes (lanes 1 and 3) and 80 U/ml of MNase for 45 minutes (lanes 2 and 4), and subjecting the solubilized samples to immunoprecipitation (CHIP) using the anti-CENP-A antibody (lanes 1 and 2) and to immunoprecipitation (CHIP) using the anti-CENP-C antibody (lanes 3 and 4). The precipitated proteins were separated by SDS-7.5% for CENP-B and CENP-C and SDS-12.5% for CENP-A, and the proteins were detected by Western blotting using ACA serum (AK). Lane M shows a marker indicating the positions of CENP-A, CENP-B, and CENP-C.

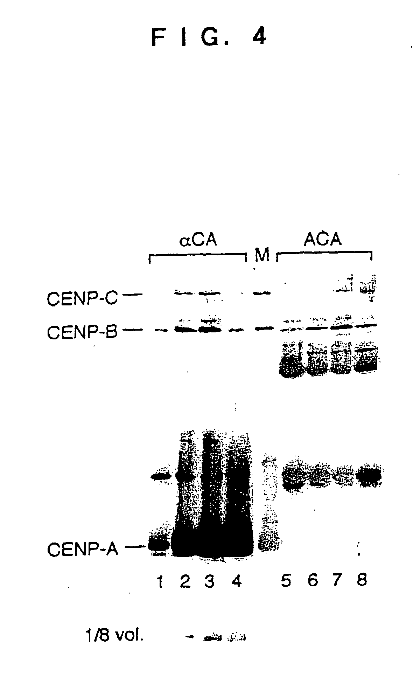

Fig. 4 is a photograph substituted for a drawing which shows the results of CENP-A, -B, and -C by Western blotting, using ACA serum, of the samples precipitated with the anti-CENP-A antibody of the present invention (αCA), and the samples obtained by further precipitating the supernatants with an ACA antibody IgG (ACA). Lanes 1 to 4 show the four samples shown in Fig. 1B, and lanes 5 to 8 show the case where each sample was precipitated with the ACA IgG. Lane M corresponds to a CENP-A/B/C marker mixture containing CENP-A, CENP-B, and CENP-C.

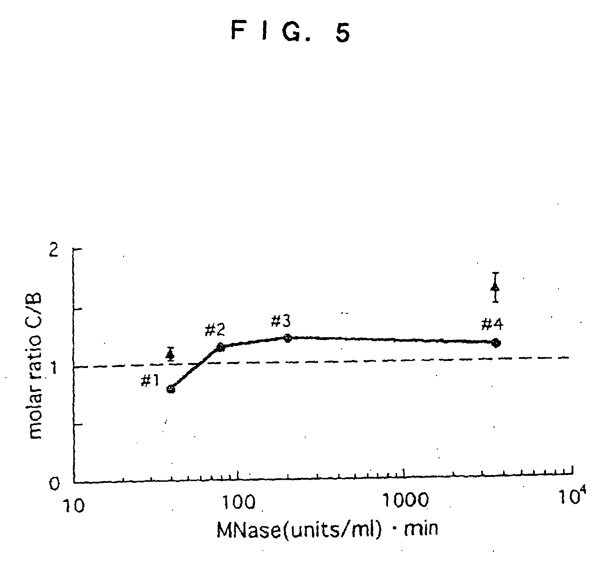

Fig. 5 is a graph showing the results obtained by calculating the molar ratios of CENP-C/B in the precipitated A/B/C complex of Fig. 4.

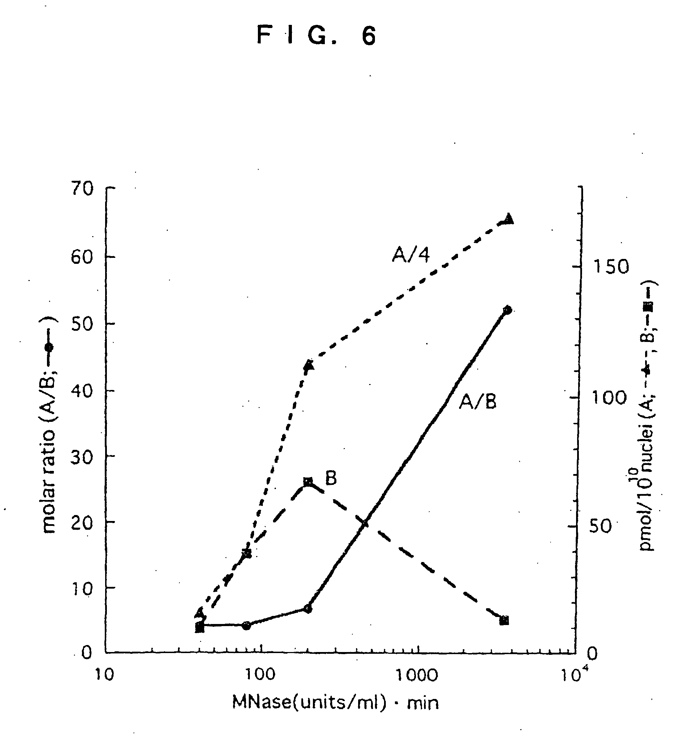

Fig. 6 is a graph showing the molar ratios of CENP-A/B and the amounts of CENP-A and CENP-B in each sample of the results of Fig. 4. The symbol ● shows the molar ratios of CENP-A/B, the symbol ▲ shows CENP-A, and the symbol ■ shows CENP-B.

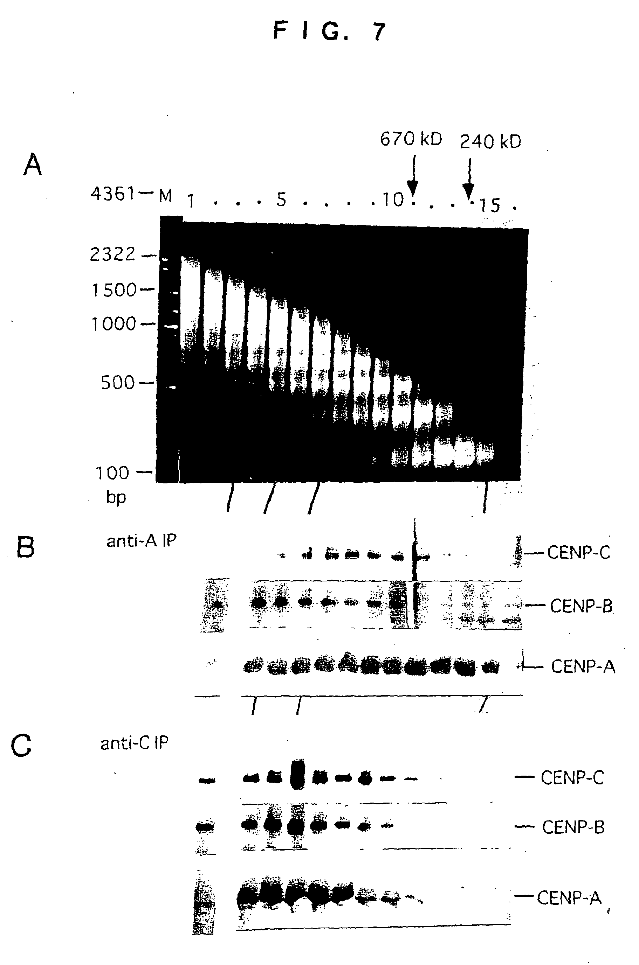

Figs. 7 are photographs substituted for drawings which show the size distributions of the centromeric chromatin immunoprecipitated with an anti-CENP-A or an anti-CENP-C antibody by 5 to 20% glycerol density gradient centrifugation.
Fig. 7A shows the distribution of the bulk chromatin of sample #3 of Fig. 1B by nucleosome DNA ladders. Figs. 7B and 7C are what were obtained by detecting the distributions of CENP-A, CENP-B, and CENP-C by the Western blotting method using ACA serum after immunoprecipitating each fraction with an anti-CENP-A antibody (Fig. 7B) or an anti-CENP-C antibody (Fig.7C).

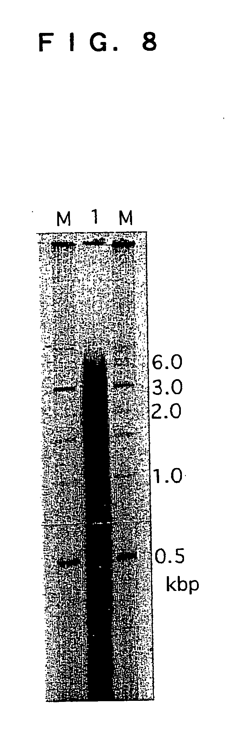

Fig. 8 is a photograph substituted for a drawing which shows the chain length distribution of the DNA contained in the centromeric chromatin isolated and purified by the anti-CENP-A antibody beads.

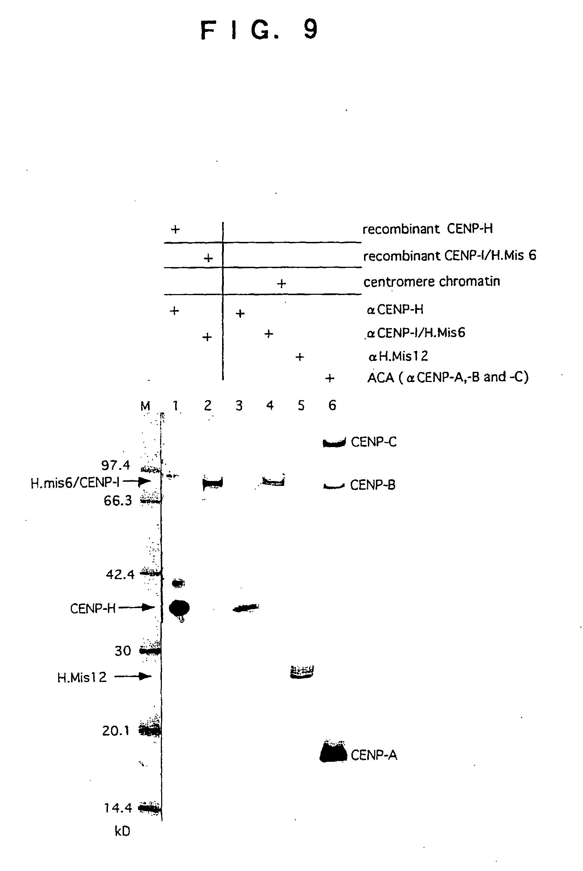

Fig. 9 is a photograph substituted for a drawing which shows that the isolated centromeric chromatin contains CENP-H, CENP-I/H. Mis-6, H. Mis-12.

CENP-H (lane 1), CENP-I/H. Mis-6 (lane 2) expressed and purified by a baculovirus system and the isolated and purified centromeric chromatins (lanes 3 to 6) were separated by 12.5% SDS-PAGE, transferred to a PVDF membrane, then immunoreaction was performed using an anti-CENP-H antibody (lanes 1 and 3), an anti-CENP-I/H. Mis-6 antibody (lanes 2 and 4), an anti-H. Mis-12 antibody (lane 5) and an ACA antibody (lane 6). A molecular weight marker was loaded on the left (lane M).

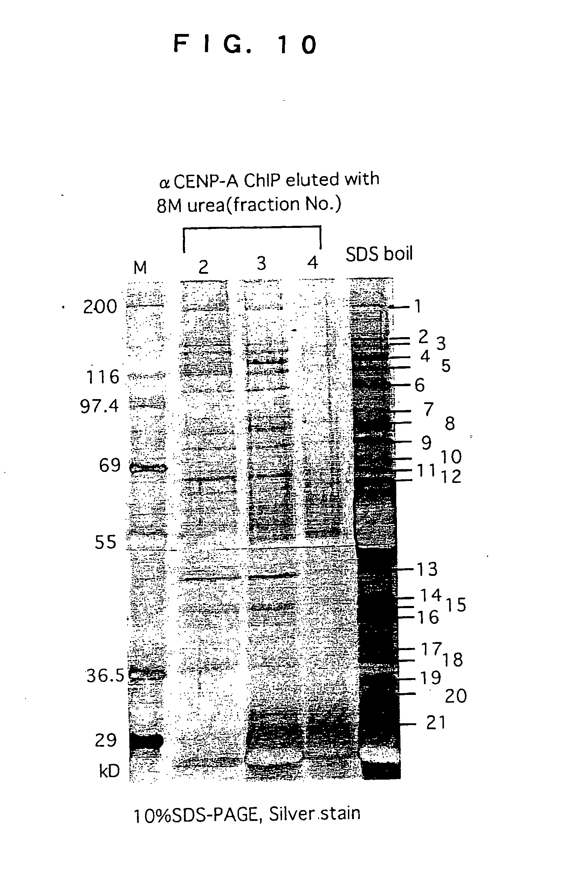

Fig. 10 is a photograph substituted for a drawing which shows the results of the proteome analysis of the substances precipitated by immunoprecipitation (CHIP), using an α-CENP-A antibody, of the centromeric chromatins (the same samples in Fig. 8 and Fig. 9) which were solubilized by MNase treatment.

Lanes 2, 3 and 4 show the numbers of the fractions obtained by eluting the precipitated substances with 8 M urea. This is silver-staining on 10% SDS-PAGE. Lane M corresponds to a molecular weight marker. The right end lane corresponds to what was obtained by adding further SDS to the beads after elution with 8 M urea and conducting boiling. The numbers on the right end show the numbers of the bands for which proteome analysis was conducted using each SDS-boil sample. The descriptions of the proteins of respective bands are described in context.

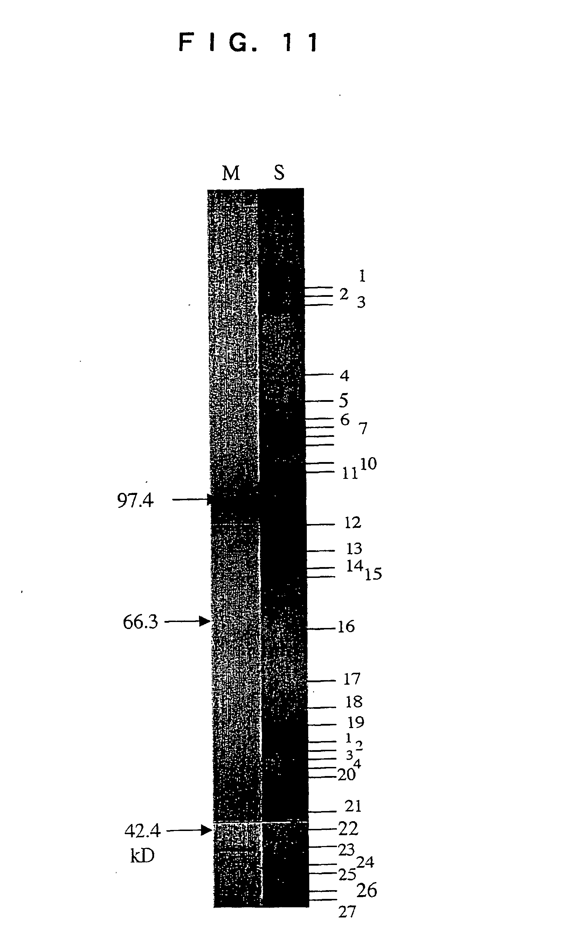

Fig. 11 is a photograph substituted for a drawing which shows the results of the Coomassie staining after protein separation, by 7.5% SDS-PAGE, of lane 3 sample eluted with 8 M urea in Fig. 10.

Lane M corresponds to a molecular weight marker. The numbers on the right end of lane S are the numbers of each band and these were subjected to proteome analysis. The descriptions of the proteins of respective bands are described in context.

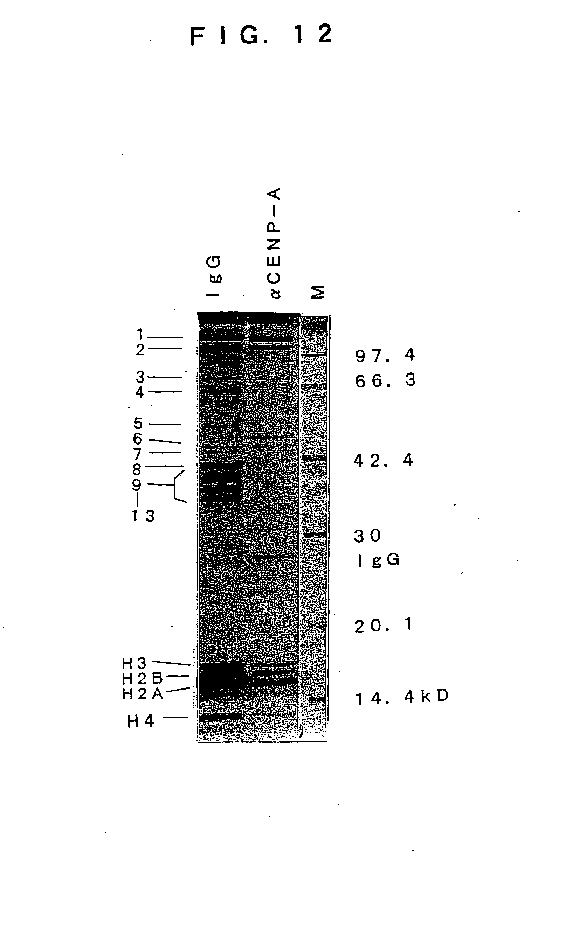

Fig. 12 is a photograph substituted for a drawing which shows the results of the immunoprecipitation using mouse IgG beads in order to compare the solubilized chromatin of the present invention. After the mouse IgG beads and the anti-CENP-A antibody beads were washed three times with a buffer containing 0.3 M NaCl, proteins were eluted stepwise with 0.6 M NaCl, 2 M NaCl and 0.1 M glycine-HCl (pH 2.5). The samples obtained by elution of the respective beads with 0.6 M NaCl were separated by 12.5% SDS-PAGE and stained with Coomassie brilliant blue (CBB). Lane IgG on the left; IgG beads, lane α-CENP-A in the center; anti-CENP-A antibody beads, lane M on the right; molecular weight marker. The numbers on the left side of the figure show the major bands in IgG lane and the descriptions of the respective bands are described in context.

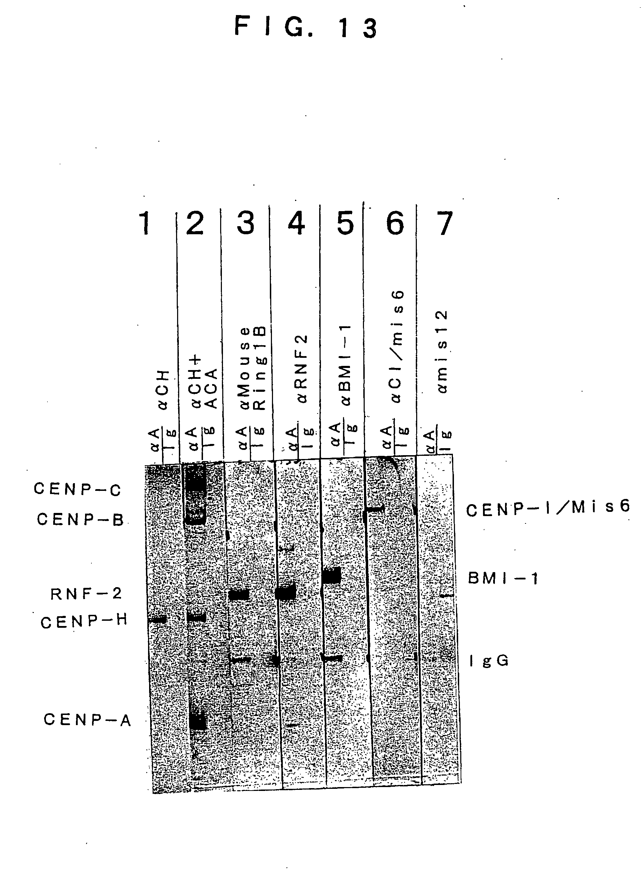

Fig. 13 is a photograph substituted for a drawing which shows the results obtained by immunostaining the samples with the antibodies shown in the upper part of Fig. 13, after the samples (αA and Ig) eluted with 0.6 M NaCl from the respective beads described in Fig. 12 were separated by 12.5% SDS-PAGE, transferred to a PVDF membrane. The left of each lane set corresponds to αCA beads samples and the right corresponds to IgG beads samples. The primary antibodies are a rabbit anti-CENP-H antibody in the lane set 1, a rabbit anti-CENP-H antibody and an ACA antibody in the lane set 2, a mouse anti-mouse Ring1B antibody (monoclonal antibody) in the lane set 3, a rabbit anti-human RNF2 antibody in the lane set 4, a mouse anti-human BMI-1 antibody in the lane set 5, a rabbit anti-CENP-I/H. Mis6 antibody in the lane set 6 and a rabbit anti-H. Mis12 antibody in the lane set 7. As the secondary antibodies, an anti-human IgG antibody (lane 2), an anti-mouse IgG antibody (lanes 3 and 5) and an anti-rabbit IgG antibody conjugated to horseradish peroxidase were used. Color development was carried out with Konica immunostain.

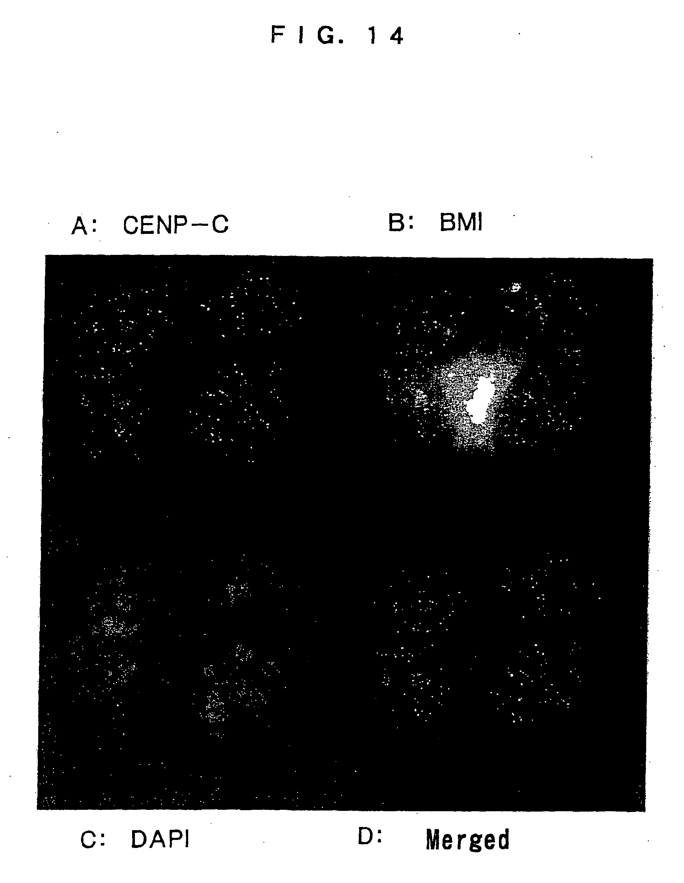

Fig. 14 is a color photograph substituted for a drawing which shows the results obtained by immunostaining HeLa cells with a BMI1 antibody and a CENP-C antibody. Fig. 14A (upper left) shows what was stained with an anti-CENP-C, Fig. 14B (upper right) shows what was stained with an anti-BMI1, Fig. 14C (lower left) shows the nucleic acid staining with DAPI, and Fig. 14D (lower right) is what was obtained by merging the three panels.

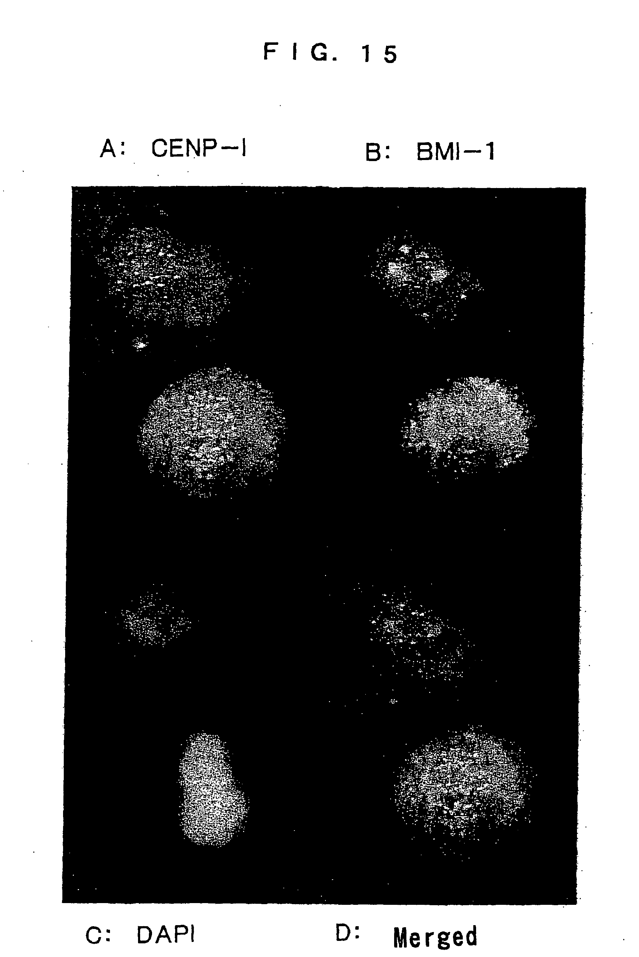

Fig. 15 is a color photograph substituted for a drawing which shows the results obtained by immunostaining HeLa cells with a BMI1 antibody and a CENP-I antibody. Fig. 15A (upper left) shows what was stained with an anti-CENP-I, Fig. 14B (upper right) shows what was stained with an anti-BMI1, Fig. 14C (lower left) shows the nucleic acid staining with DAPI, and Fig. 14D (lower right) is what was obtained by merging the three panels.

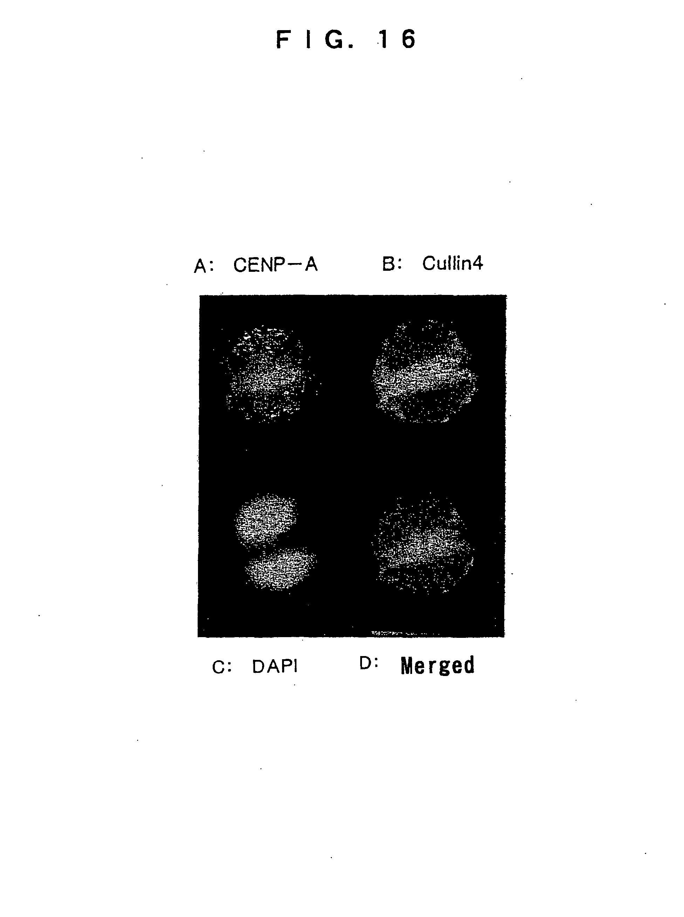

Fig. 16 is a color photograph substituted for a drawing which shows the results obtained by immunostaining HeLa cells with an antibody against Cullin 4 corresponding to the 14^{th} band of Fig. 11. Fig. 16A (upper left) shows what was stained with an anti-CENP-A, Fig. 16B (upper right) shows what was stained with an anti-Cullin 4, Fig. 16C (lower left) shows the nucleic acid staining with DAPI, and Fig. 16D (lower right) is what was obtained by merging the three panels.

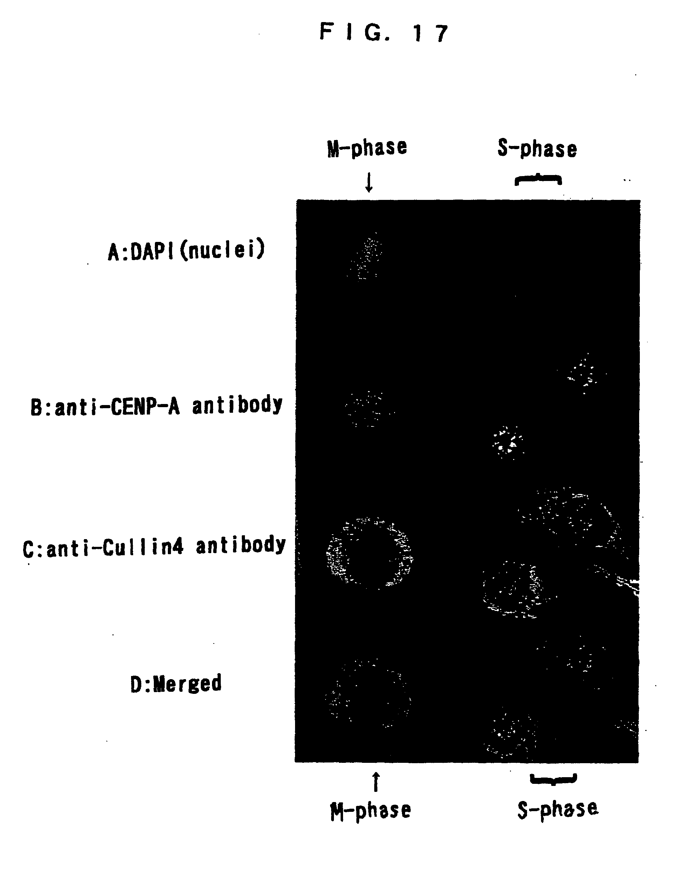

Fig. 17 is a color photograph substituted for a drawing which shows the results obtained by staining M-phase (left side of each figure) and S-phase (right side of each figure) cells by the same method as in Fig. 16. Fig. 17 is composed of 4 photographs of M-phase (left side of each figure) and S-phase (right side of each figure) respectively, and 8 photographs in total, and A, B, C and D from the top. Fig. 17A (the uppermost row) shows the staining of DNA with DAPI, Fig. 17B (the second row from the top) shows what was stained with an anti-CENP-A, Fig. 17C (the third row from the top) shows what was stained with an anti-Cullin 4, and Fig. 17D (the lowermost row) shows what was obtained by merging the three panels, respectively.

### Best Mode for Carrying Out the Invention

The present inventors had investigated a method of simplifying the selection and establishment of a hybridoma cell as much as possible in a method of producing a monoclonal antibody by a cell fusion method and found that, by using, as a myeloma cell, P3-X63-Ag8.653 cell, which is a mouse myeloma cell with a high fusion efficiency and using GIT medium (Nihon Pharmaceutical Co., Ltd.) that requires only a small amount of fetal bovine serum as a cell culture medium, a selection can be carried out with equal to or less than half of a conventional medium area at the first screening. For example, as a 96-well plate, a half-well plate that is about half of it can be used and the whole well can be observed in a single sight when the appearance number of clones is confirmed by a microscope, therefore, a rapid screening became possible.

The present inventors had difficulty in producing an antibody that reacted with a CENP-A protein effectively even though an antibody was produced using the whole length of a CENP-A protein because the CENP-A protein was a difficult protein to specify an active site having antigenicity. Therefore, to produce an antibody that reacts with the CENP-A protein effectively, antibodies were produced using various kinds of CENP-A protein fragments. In other words, using a fragment composed of the amino acid sequence of 3- to 19-positions of a human CENP-A protein (#3-19), a fragment composed of the amino acid sequence of 12- to 25-positions of the human CENP-A protein (#12-25), a fragment composed of the amino acid sequence of 27- to . 40-positions of the human CENP-A protein (#27-40), a fragment composed of the amino acid sequence of 39- to 53-positions of the human CENP-A protein (#39-53) and a fragment composed of the amino acid sequence of 126- to 140-positions of the human CENP-A protein (#126-110) as respective antigens, a screening of hybridoma cells producing a monoclonal antibody was conducted.

As a result, an anti-CENP-A peptide (#3-19) antibody, an anti-CENP-A peptide (#27-40) antibody and an anti-CENP-A peptide (#126-140) antibody were found to be extremely effective antibodies. In particular, the anti-CENP-A peptide (#3-19) antibody was found to give extremely good results in the Western blotting method, the indirect immunofluorescent staining method and the immunoprecipitation method.

As a method of producing antibodies against lots of these peptide fragments, the above-mentioned method of producing an antibody of the present invention was also found to be effective.

Similarly, the anti-CENP-B antibody and the anti-CENP-C antibody were produced. In other words, the antibodies were produced using, as an antigen, a recombinant peptide, which had been obtained by expressing a peptide containing half the amino acid sequence of the N-terminal side of a CENP-B protein by an E. coli expression system, or using, as an antigen, a recombinant peptide, which had been obtained by expressing a peptide containing half the amino acid sequence of the N-terminal side of a CENP-C protein by a baculovirus expression system.

Next, the present inventors carried out the following experiments to confirm that the obtained antibodies of the present invention reacted effectively and to demonstrate their application examples.

First, the present inventors examined the conditions in which centromeric chromatin is solubilized from the nuclei of HeLa cells. The results of the examination for solubilization by NaCl are shown in lanes 1 to 3 of Fig. 1A. The result obtained by treating the nuclei with 0.3 M NaCl is shown in lane 1 of Fig. 1A. In this condition, none of CENP-A, -B, and -C was confirmed to be eluted into the soluble fraction. The results of the supernatant and the precipitate by 0.6 M NaCl treatment are shown in lane 2 and lane 3 of Fig. 1A, respectively. As a result, the solubilization of CENP-B and CENP-C was observed (lane 2 of Fig. 1A), however, the solubilization of CENP-A was not observed. Next, the results of the supernatant and the precipitate in the case where the nuclei were digested with micrococcal nuclease (MNase) in 0.3 M NaCl are shown in lane 4 and lane 6 of Fig 1A, respectively. As a result, it was found that, in the case of MNase treatment, 30 to 50% was solubilized in any of CENP-A, -B, and -C. In addition, in the Western analysis, each band was confirmed as the centromere protein by the fact that the respective bands were removed when ACA-beads were added to the sample (lane 5 of Fig. 1A).

From these results, it was found that centromeric chromatin containing CENP-A can be eluted into a soluble fraction by MNase digestion in the same manner as bulk chromatin.

Next, kinetics of CENP-A elution was carried out to find out the degree of released centromeric chromatin after the digestion of the nuclear pellet of HeLa cells with micrococcal nuclease (MNase). The isolated HeLa cells were digested with MNase to various degrees (40 to 3,600 U/ml x min). The results are shown in Fig. 1B with a photograph substituted for a drawing. Fig. 1B shows the size distribution of the DNA of each sample after MNase digestion. HeLa nuclei were digested with MNase at 37°C, DNA extracted from the soluble fraction was electrophoresed on a 1% agarose gel, and the results of the DNA detection by ethidium bromide staining are shown. The conditions of MNase digestion are as follows. Lane 1 was 20 U/ml for 2 minutes (40 U/ml x min., hereinafter referred to as sample #1), lane 2 was 20 U/ml for 4 minutes (80 U/ml x min., hereinafter referred to as sample #2), lane 3 was 40 U/ml for 5 minutes (200 U/ml x min., hereinafter referred to as sample #3), and lane 4 was 80 U/ml, for 45 minutes (3,600 U/ml x min., hereinafter referred to as sample #4).

In addition, the results of the fractionation by SDS-PAGE and the detection by Coomassie brilliant blue (CBB) staining of the bulk chromatin proteins in a soluble fraction and an insoluble fraction after MNase digestion are shown in the upper photograph of Fig. 1C, which is substituted for a drawing. At this time, the relative amount of the bulk chromatin was measured by the band intensity of histone H4, and the relative amount of the centromeric chromatin was measured by the amount of CENP-A. The results of the identification of CENP-A by immunostaining with the use of an ACA antibody after transferring the fractionated proteins to a membrane are shown in the lower photograph of Fig. 1C, which is substituted for a drawing. Lanes 1 to 4 of Fig. 1C correspond to the samples from the supernatant, lanes 5 to 8 correspond to the samples from the pellet and lane M shows the position of CENP-A.

These results indicate that centromeric chromatin containing CENP-A can be solubilized by MNase digestion in the same manner as bulk chromatin.

Next, to investigate the structure and the composition of a centromeric chromatin complex containing CENP-A, a chromatin immunoprecipitation (CHIP) with the anti-CENP-A monoclonal antibody of the present invention was carried out. Chromatin containing a trinucleosome and a larger nucleosome was recovered by 5 to 20% glycerol gradient sedimentation of sample #3 of Fig. 1B and immunoprecipitated with an anti-CENP-A, -B and -C. It was found that α-satellite DNA had been concentrated by a slot-blot analysis (data not shown). The results are shown in Fig. 2. Fig. 2 shows the distribution of the DNA sequence contained in the chromatin obtained by the immunoprecipitation of α-satellite DNA containing a CENP-B box with an anti-CENP-A, an anti-CENP-B or an anti-CENP-C antibody. The IP of the three columns on the right side of Fig. 2 shows the distribution of the determined nucleotide sequence of the DNA fragments immunoprecipitated with the anti-CENP-A antibody (referred to as αCA in Fig. 2), the anti-CENP-B antibody (referred as αCB in Fig. 2) or the anti-CENP-C antibody (referred to as αCC in Fig. 2) and cloned into Topo vector (Invitrogon). Equal to or more than 80% of the cloned fragments were longer than 500 bp. "Before IP" on the left side of Fig. 2 shows clones produced from the solubilized bulk chromatin before immunoprecipitation. The black bars show the number of clones with α-satellite DNA that contains a CENP-B box. The open bars show the number of clones other than these. These clones have unique sequences, containing no other repeated sequence, even α-satellite DNA without a CENP-B box.

As summarized in Fig. 2, α-satellite DNA containing a CENP-B box could be concentrated by CHIP using each of the three antibodies. It has already been shown that CENP-B preferentially binds to the CENP-B box. It is therefore to be expected that CHIP using the anti-CENP-B antibody would precipitate the α-satellite DNA with a CENP-B box (20 clones; 31% of total clones), and the DNA from the other 43 clones (69%) is considered to be background, which may reflect the quality of this anti-CENP-B monoclonal antibody. The CHIP using the anti-CENP-C antibody significantly concentrated the CENP-B box of α-satellite DNA (26%), while the CHIP using the anti-CENP-A antibody precipitated the α-satellite DNA containing a CENP-B box at a very high rate (76%). Through all CHIP analyses, an α-satellite DNA fraction without a CENP-B box was not recovered. These results suggest that a centromeric chromatin complex containing CENP-A, -B and -C is formed selectively in α-satellite DNA (αI-type sequence) containing a CENP-B box in a HeLa cell.

Next, coprecipitation of the solubilized chromatin in samples #3 and #4 shown in Fig. 1B in the immunoprecipitation was examined. The results are shown in Figs. 3 with photographs substituted for drawings. Fig. 3A shows that the produced anti-CENP-A antibody and anti-CENP-C antibody stain the centromere in M phase chromosome with extremely high specificity. Using these antibodies, in Fig. 3B, the analysis was carried out by Western blotting using ACA serum after 7.5% (the upper panel of Fig. 3B) or 12.5% (the lower panel of Fig. 3B) SDS-PAGE following the immunoprecipitation of the solubilized chromatin with the anti-CENP-A antibody (lanes 1 and 2) or the anti-CENP-C antibody (lanes 3 and 4). Lanes 1 and 3 show the case of using sample #3, and lanes 2 and 4 show the case of sample #4. In sample #3, CENP-B and -C (lane 1) or CENP-A and -B (lane 3) are shown to be co-precipitated. However, in sample #4 that was subjected to a strong MNase digestion, the co-precipitated centromere protein was greatly decreased (lane 2 and 4). These results show that, in 0.3 M NaCl, CENP-B and CENP-C interact with a CENP-A nucleosome via DNA.

It is difficult to detect and quantitate CENP-B and CENP-C in a bulk chromatin fraction directly by Western blotting because of high background. Therefore, the amounts of CENP-B and CENP-C which interact with a CENP-A nucleosome were measured by two-step immunoprecipitation using an anti-CENP-A antibody and ACA serum. As a result, it was demonstrated that the newly produced anti-CENP-A antibody of the present invention can precipitate CENP-A chromatin quantitatively. Each of the four samples described in Fig. 1B was immunoprecipitated with the anti-CENP-A antibody, and then the supernatant was precipitated with ACA serum. The both sets of the precipitates (αCA and ACA) were analyzed by Western blotting with ACA.

The results are shown in Fig. 4 with a photograph substituted for a drawing. Fig. 4 shows the results of contents of CENP-A, -B and -C in the CENP-A/B/C chromatin complex precipitated with the anti-CENP-A antibody by Western blotting using ACA serum. The four MNase digested samples shown in Fig. 1B were immunoprecipitated using the anti-CENP-A antibody (lanes 1 to 4: 12 µl samples of αCA). The supernatants were subsequently immunoprecipitated by an ACA antibody (lanes 5 to 8: 12 µl samples of ACA), and residual CENPs were detected using ACA serum. The precipitated proteins were separated by 12.5% SDS-PAGE, blotted on a PVDF membrane and immunostained using ACA serum. Because CENP-A in the αCA sample (lanes 2 to 4) was excessive, 1.5 µl of the samples were also immunostained and are shown below. The broad smear bands between CENP-A and CENP-B seen in the both samples are IgG released from the antibody Sepharose beads. Lane M corresponds to 4 µl of a CENP-A/B/C marker mixture containing CENP-A (61 fmol/µl), CENP-B (23 fmol/µl) and CENP-C (25 fmol/µl) and the molar ratio of A/B/C is 2.65:1:1.09.

As shown in Fig. 4 (lanes 1 to 4), CENP-B and CENP-C were co-precipitated with CENP-A. Fig. 4 (lanes 5 to 8) shows that CENP-A chromatin was almost removed from the supernatant solution, and CENP-C also disappeared by the early digestion (Fig. 4, lanes 5 and 6), however 40 to 50% of the solubilized CENP-B apparently remained in the supernatant after the CENP-A chromatin removal (Fig. 4, lanes 5 and 6). When the precipitate shown in Fig. 4 (lane 3) was further digested by MNase or treated with 0.6 M NaCl, CENP-B and CENP-C were released into the supernatant (data not shown). From these results, it was concluded that almost all of solubilized CENP-C and about half of the solubilized CENP-B formed a complex with a CENP-A nucleosome via DNA.

As shown in Fig. 4, the amounts of CENP-B and CENP-C increased (lanes 1 to 3), reached a maximum (lane 3) and then decreased as DNA cleavage progressed (lane 4). On the other hand, the amount of CENP-A continued to increase. These results indicated that the MNase digestion of HeLa cells caused two events. In other words, first, it releases the CENP-A/B/C complex into the soluble fraction, then removes CENP-B and/or CENP-C from the complex. Therefore, the molar ratio of CENP-A/B/C in the released complex by weak MNase treatment (Fig. 1B, #1 and #2) may represent the molar ratio in the pre-kinetochore CENP-A/B/C complex. To measure the stoichiometry of the pre-kinetochore chromatin complex, an A/B/C mixture with a known concentration was produced using a recombinant protein expressed by a baculovirus system. It was confirmed that the reactivity of these recombinant proteins against ACA serum was the same as that of the proteins purified from HeLa cells (data not shown): Although the molar ratio of CENP-C/B of the standard mixture in lane M of Fig. 4 was 1.09, the intensity of the CENP-C band was a little weaker than that of CENP-B because the transfer efficiency of CENP-C to a membrane was low. The results of CENP-C/B molar ratio calculation in lanes 1 to 4 of Fig. 4 in consideration of this are shown in Fig. 5. Fig. 5 shows the calculated values of CENP-C/B molar ratio in the precipitated A/B/C complex. The intensities of CENP-C and CENP-B in lanes 1 to 4 of Fig. 4 were measured by an NIH image analysis software, and using the intensity value of the marker mixture (C/B molar ratio = 1.09) in lane M of Fig. 4, the intensity ratios of CENP-C/B were converted into molar values. The C/B molar ratios of samples #1 and #4 were corrected using 3 µl of the marker mixture, and the corrected values are shown (black triangle symbol).

As a result, they were shown to be constant and about 1. On the other hand, the intensity ratio of CENP-A/B practically represented its molar ratio. Based on these data, the kinetics of the CENP-A/B molar ratio is shown in Fig. 6. Fig. 6 shows the molar ratio of CENP-A/B and the amounts of CENP-A and CENP-B in each sample. A/B intensity ratios in sample #1 (lane 1 of Fig. 4) and in samples #2 to 4 (3 µl of each sample was used for Western blotting) were measured and plotted as A/B molar ratios (-●-). The A/B molar ratios were 4.26 in sample #1 and 4.10 in sample #2. The amounts of CENP-A and CENP-B were calculated by comparing the band intensities with those of the marker proteins. CENP-A is shown with the symbol ▲, CENP-B is shown with the symbol **■** and the scale is shown in the right axis. One-fourth of CENP-A was plotted against the strength of MNase digestion (U/ml x min.).

As a result, CENP-C/B ratio and CENP-A/B molar ratio at early digestion (samples #1 and #2) were mostly constant and 1.1, 0.1 and 4.2, 0.4 respectively. The amounts of CENP-A and CENP-B in each sample in 10¹⁰ nuclei equivalent were described in Fig. 6. From these results, it could be concluded that the average molar ratio of CENP-A:B:C in the pre-kinetochore chromatin complex is about 4:1:1.

CENP-A/B/C chromatin can be cut into a 3 to 4mer nucleosome by MNase digestion.

Next, to examine how short the CENP-A/B/C chromatin can be cut into while maintaining the complex, the bulk chromatin solubilzed by MNase digestion with 60 U/ml at 37°C for 15 minutes was fractionated based on molecular weight by 5 to 20% glycerol density gradient centrifugation. Each fraction was immunoprecipitated using an anti-CENP-A or an anti-CENP-C antibody and CENP-A, -B and -C were detected with ACA serum after SDS-PAGE. The results are shown in Figs. 7 with photographs substituted for drawings. Fig. 7A shows the distribution of the bulk chromatin with DNA ladders contained in the chromatin. Fig. 7B shows the CHIP of each fraction by the anti-CENP-A antibody, and Fig. 7C shows the CHIP of each fraction by the anti-CENP-C antibody. In the CHIP by the anti-CENP-C antibody, the intensity distribution of CENP-A, -B and -C corresponds (Fig. 7C). This strongly suggests that, by CHIP using anti-CENP-C antibody, the CENP-A/B/C complex is mainly immunoprecipitated. According to the results of Figs. 7B and C, the peak of the CENP-A/B/C complex is located in fraction #7. This fraction corresponds to the precipitation position of 5 to 6mer nucleosome according to the DNA ladders in Fig. 7A. The molecular weight of a mononucleosome corresponds to about 240 kD. CENP-B + CENP-C is about 220 kD and corresponds to the molecular weight of the mononucleosome. Accordingly, mononcleosome + CENP-B + CENP-C becomes at least 460 kD or more. Therefore, if the molecular weights of CENP-B and CENP-C are taken into account, fraction #7 is to correspond to a CENP-A/B/C complex of 3 to 4mer nucleosome. From these results, it is possible to conclude that CENP-A/B/C chromatin can be cut until 3 to 4mer nucleosome by MNase digestion.

As mentioned above, it was found that the CENP-A antibody of the present invention binds to a CENP-A protein extremely specifically and extremely effective for an analysis of a centromere or the like. In addition, the CENP-A antibody of the present invention is effective in screening or diagnosis of chromosomal aberration. Because the primary factor to determine a centromere region is the formation of a nucleosome containing a CENP-A protein (CENP-A nucleosome), the CENP-A antibody of the present invention is an extremely effective antibody to detect and identify a region to form a centromere as a marker to indicate the centromere region. Specifically, for example, there is a disease in which a centromere is formed in a region other than the normal centromere region, and for such a disease, the antibody of the present invention can detect abnormality of the centromere surely and highly sensitively.

Next, the present inventors performed the identification of a known centromere protein contained in centromeric chromatin other than CENP-A, -B and -C. By the method of the present invention, it was demonstrated that the CENP-A/B/C chromatin complex can be eluted into a soluble fraction while retaining the integrity by treating the isolated nuclei of HeLa cells with micrococcal nuclease relatively weakly in the presence of 0.3 M NaCl, and further isolated and purified by using anti-CENP-A antibody beads. These results strongly suggest the possibility that another centromere protein is further contained in the isolated CENP-A/B/C chromatin as a constitutive factor.

Therefore, CENP-H and CENP-I/human Mis-6 genes, which were recently reported, were obtained. The CENP-H gene was obtained from Dr. Todokoro (RIKEN). The CENP-I/H.Mis-6 was obtained from HeLa cDNA library by cloning after the PCR amplification. A target protein was purified by expressing each protein by the baculovirus system, separating proteins by SDS-PAGE and cutting out a band. The antibodies were produced by immunizing a rabbit for an anti-CENP-H antibody and a rat for an anti-CENP-I antibody (contracted to Oriental Yeast, Co., Ltd.). The anti-human Mis-12 antibody was obtained from Dr. Mitsuhiro Yanagida.

The nuclei of HeLa cells were isolated (5 X 10⁹), and the CENP-A/B/C chromatin complex was solubilized by the treatment with 6 U/ml MNase at 37°C for 30 minutes in the presence of 0.3 M NaCl and isolated and purified by using the anti-CENP-A antibody beads. The length of the DNA at this time was 0.3 to 5 kbp. The results are shown in Fig. 8 with a photograph substituted for a drawing. Lane M of Fig. 8 shows a marker.

Then, proteins were separated by SDS-PAGE of the isolated and purified centromeric chromatin and the identification of the target proteins was attempted by Western blotting with the use of the respective antibodies. The results are shown in Fig. 9 with a photograph substituted for a drawing. Lane M of Fig. 9 indicates a marker and lanes 1 to 6 indicate that the one marked with + symbol shown above each lane was used. The used ones are "recombinant CENP-H", "recombinant CENP-I/Human Mis6", "centromeric chromatin", "CENP-H antibody", "CENP-I/Human Mis6 antibody", "Human Mis12 antibody" and "ACA" from the top. As indicated in Fig. 9, it was found that the isolated centromeric chromatin contains CENP-A, -B and -C (lane 6), and further contains CENP-H (lane 3), CENP-I/human Mis6 (lane 4) and human Mis-12 (lane 5) simultaneously.

Here, the meaning of using an anti-CENP-A monoclonal antibody in chromatin immuno-precipitation (CHIP) is explained.

As the above-mentioned experimental results show, it was revealed that the anti-CENP-A monoclonal antibody of the present invention can isolate and purify centromeric chromatin while well retaining the integrity by the CHIP method. The conventional CHIP method adopted the solubilization method in which a complex was crosslinked using formaldehyde and then DNA was cut by sonication or MNase treatment, however, an analysis of proteins was difficult by this method. Therefore, in the present invention, it was found that without conducting formaldehyde treatment, the solubilization can be performed by MNase treatment under a very mild condition.

There are two important points in the present invention. The first point is an anti-CENP-A monoclonal antibody with high specificity, and the second point is the method of solubilizing centromeric chromatin while retaining the integrity. The present invention is to provide an anti-CENP-A monoclonal antibody and a method of isolating centromeric chromatin while retaining the integrity as a method of using the same.

The solubilization method of the present invention is what enables solubilization by MNase treatment under a very mild condition. The concentration of the MNase to be used is 1 U/ml to 500 U/ml, preferably 10 U/ml to 100 U/ml. The treatment time varies depending on the condition of the centromeric chromatin to be solubilized, the concentration of the MNase to be used or the degree of the desired solubilization, however, it is generally 1 minute to 100 minutes, preferably 1 minute to about 50 minutes. The treatment temperature is not particularly restricted as long as it can maintain the activity of MNase, however, a temperature around body temperature is preferable.

This treatment can be carried out in an appropriate buffer solution and, for example, WB buffer solution can be used as in the description of Examples mentioned below. NaCl, CaCl₂ or the like can be added to a buffer solution as needed.

Incidentally, in the article (Satoshi Ando, et al., Molecular Cellular Biol., 22(7), 2229-2241 (2002)) published by the present inventors after the priority application of the present application, a method of isolation of centromeric chromatin while retaining the integrity is described by analyzing, in detail, the dynamics of centromeric chromatin by MNase treatment.

Examples of analyzing centromeric chromatin that was solubilized and isolated by the method of the present invention are shown in Fig. 10 and Fig. 11 with photographs substituted for drawings. Fig. 10 shows what was obtained by immunoprecipitating the solubilized centromeric chromatin with a CENP-A antibody, eluting samples with 8 M urea (lanes 2 to 4) or subsequently subjecting to heating in the presence of SDS (SDS-boil); resolving the samples by 10% SDS-PAGE, and conducting silver-staining. The numbers on the right of Fig. 10 show the bands of proteins detected by the silver-staining method for SDS-boil samples, and proteome analysis was conducted for these. The results of the proteome analysis obtained by analyzing the bands of proteins indicated by the numbers on the right of Fig. 10 are as follows.
1 is an unknown protein,
2 is an unknown protein,
3 shows "no hit" in which a corresponding protein could not be found by a search,
4 shows CENP-C,
5 shows mitotic Kinesin-like 1,
6 shows mitotic Kinesin-like 1,
7 shows mitotic Kinesin-like 1,
8 shows a zinc finger protein homologous to mouse Zfp91,
9 shows rac GTPase activating protein 1,
10 shows rac GTPase activating protein 1,
11 shows rac GTPase activating protein 1,
12 shows "no hit" in which a corresponding protein could not be found by a search,
13 shows rectachrome 1 (Human Polycomb 3),
14 shows polycomb complex protein Bmi-1 and polyhomeotic protein 2 (PHP-2),
15 shows human Ring finger protein 2 (mouse Ring 1B) and polycomb complex protein Bmi-1,
16 shows human Ring finger protein 2 (mouse Ring 1B) and macro-histone H2A1.1,
17 shows "no hit" in which a corresponding protein could not be found by a search,
18 shows fibrillarin; 34 kD nucleolar scleroderma protein,
19 shows lamin A precursor,
20 shows B cell antigen receptor, and
21 shows histone H4.

Fig. 11 is a photograph substituted for a drawing which shows the results obtained by immunoprecipitating the solubilized bulk chromatin with a CENP-A antibody by the method of the present invention, eluting the isolated centromeric chromatin complex with 8 M urea (the same sample as in lane 3 of Fig. 10), and separating the eluted proteins by 7.5% SDS-PAGE. The numbers on the right of Fig. 11 show the proteins detected by proteome analysis for the respective bands. The results obtained by analyzing the bands of proteins indicated by the numbers on the right of Fig. 11 are as follows.
1 is an unknown protein,
2 is an unknown protein,
3 is an unknown protein,
4 is an unknown protein,
5 shows SWI/SNF related protein,
6 shows CENP-C,
7 shows DNA damage binding protein,
8 is an unknown protein,
9 shows "no hit" in which a corresponding protein could not be found by a search,
10 shows "no hit" in which a corresponding protein could not be found by a search,
11 shows kinesin-like protein 5 (kinesin-like 5),
12 is and shows an unknown protein,
13 shows CENP-B,
14 shows CENP-I/hMis-6, Heat shock 70 kD protein 5 and Cullin 4A,
15 shows RacGAP-1,
16 is an unknown protein,
17 shows HDAC-1,
18 is an unknown protein,
19 is an unknown protein,
19-1 is an unknown protein,
19-2 is an unknown protein, or shows Rfp-1b and BMI-1
19-3 shows Rfp-2, BMI-1, Zfp-91 and β-actin,
19-4 shows histone macro H2A2.1,
20 shows rectachrome (Hpc-3) and Rfp-1,
21 shows RuvB-like protein 2, DEAD-box protein and hArpNbeta/BAF 53,
22 is an unknown protein,
23 is an unknown protein,
24 shows CENP-H (206),
25 shows CENP-H (109),
26 shows CENP-H (332), and
27 shows SoxLZ/Sox 6 leucine zipper binding protein and Chromobox homolog protein 8 (Chromobox homolog 8).

CENP-A, -B, -C, -H, and -I/hMis-6 identified by Western analysis in Fig. 10 and Fig. 11 could be identified as amino acid sequences. Further, regarding the unknown proteins and the like, proteome analysis using the isolated centromere chromatin is being continued. Furthermore, equal to or more than 60 proteins has been identified to date (as an example, see Fig 10).

Further, it was checked whether or not a protein group immunoprecipitated with the anti-CENP-A antibody beads contained a protein which had been nonspecifically adsorbed to an IgG antibody. To be more precise, mouse IgG (purchased) beads were added to the chromatin solubilized by the method of the present invention, and mixed at 4°C for one whole day and night. Then, immunoprecipitation of the supernatant was conducted with the anti-CENP-A antibody beads. Proteins were eluted from the respective samples and proteome analysis was conducted by the same method as described above to identify genes. The results are shown in Fig. 12 with a photograph substituted for a drawing. The lane on the left in Fig. 12 corresponds to the results of the mouse IgG, and the numbers of 1 to 13 indicate the detected protein bands. From the results of the proteome analysis of these detected proteins, band 1 was ATP-dependent RNA helicase (1586; this value shows the degree of homology between the determined peptide and the database. hereinafter same as this), band 2 was a transcription control protein 80 (816) and double-stranded RNA-specific adenosine deamylase (699), band 3 was DEAD/H box polyputide 3 (1022), band 4 was growth regulated nuclease 68 protein (965), band 6 was BAF 53 (91) and actin-like protein 6 (222), band 7 was interleukin enhancer binding factor 2 (371), band 8 was histone macro-H2A1.1. (336) and actin (193), and bands 9 to 13 were heterogeneous nuclear ribonucleoproteins. The lane on the right in Fig. 12 corresponds to the case of using α-CENP-A. Lane M on the right corresponds to a marker. As a result, the spectra of proteins recovered from the anti-CENP-A beads were almost identical to the results obtained in Fig. 10 and Fig. 11. On the contrary, the spectra of proteins bound to the mouse IgG beads were completely different (see Fig. 12). The overlapping proteins of both were only histone H1, core histones (H3, H2A, H2B and H4), actin and histone macro-H2A1.1. The results show that the proteins recovered by the anti-CENP-A beads are specific to centromeric chromatin.

Next, antibodies against RNF 2, which is a constitutive factor of a polycomb complex detected by proteome analysis, and BMI1, which is known as a gene associated with a cancer, were obtained. And it was confirmed that these proteins were contained in the isolated CENP-A/B/C complex by the Western blot method. The results are shown in Fig. 13 with a photograph substituted for a drawing. The left of each lane set 1 to 7 of Fig. 13 corresponds to the αCA beads sample and the right corresponds to the IgG beads sample. The primary antibodies of the respective lane sets were a rabbit anti-CENP-H antibody in the lane set 1, a rabbit anti-CENP-H antibody and an ACA antibody in the lane set 2, a mouse anti-mouse Ring1B antibody (monoclonal antibody) in the lane set 3, a rabbit anti-human RNF2 antibody in the lane set 4, a mouse anti-human BMI-1 antibody in the lane set 5, a rabbit anti-CENP-I/H. Mis6 antibody in the lane set 6 and a rabbit anti-H. Mis12 antibody in the lane set 7. As the secondary antibodies, an anti-human IgG antibody (lane 2), an anti-mouse IgG antibody (lanes 3 and 5), an anti-rabbit IgG antibody conjugated to horseradish peroxidase were used, respectively. Color development was carried out with Konica immunostain. CENP-H in the lane set 1, and CENP-H and CENP-A, -B, -C in the lane set 2 are detected. CENP-A is not normally eluted with 0.6 M NaCl, however, since a large amount of CENP-A exists, a part of it has been leaked. Actually, a large amount of CENP-A has been eluted with 2 M NaCl at pH 2.5. In the lane sets 3 and 4, bands with the same mobility have been detected. Human RNF2 has been identified in both lane sets since it is a mouse Ring1B homolog and has 99% homology. It is concluded that the protein detected in the lane sets 3 and 4 is RNF-2. In the lane set 5, BMI-1 has been detected as three bands. In the lane set 6, CENP-I/H. Mis6 has been detected. In the lane set 7, though it is very faint, the band has been detected just below the IgG band. In the lane sets 3 and 5, the bands, which appear below the RNF2 and BMI-1, respectively, are the mouse IgG antibody leaked from the beads. In the lane sets 3 and 5, since the anti-mouse IgG are used, it has been stained. In other lane sets, because it is the anti-IgG antibody against a different animal species, it has been detected as a very faint band. Fig. 9 shows the results of the Western analysis against proteins eluted with 8 M urea, and CENP-A, -B, -C, -H, -I/H. Mis6 and H. Mis12 have been reproducibly identified. Here, RNF2 and BMI-1 have been further identified.

In addition, HeLa cells were immunostained using the same antibodies. The results are shown in Fig. 14 and Fig. 15 with photographs substituted for drawings. Fig. 14A (upper left) shows what was stained with the anti-CENP-C, Fig. 14B (upper right) shows what was stained with the anti-BMI1, Fig. 14C (lower left) shows the nucleic acid staining with DAPI, and Fig. 14D (lower right) is what was obtained by merging the three panels. Fig. 15A (upper left) shows what was stained with the anti-CENP-I, Fig. 15B (upper right) shows what was stained with the anti-BMI1, Fig. 15C (lower left) shows DAPI, and Fig. 15D (lower right) is what was obtained by merging the three panels. As a result, a large number of dots were observed in the nuclei for RNF2, however, it could not be determined whether or not there were dots overlapping with the centromere (data not shown). On the contrary, BMI1 was observed as dots in the nuclei in the same manner, and these dots completely overlapped with the dots shown by CENP-C, CENP-H or CENP-I/H. Mis6. Therefore, it has been proved for the first time that the BMI1 protein is a constitutive protein of a centromere. It is known that a polycomb complex has a function to suppress transcription of a certain gene by heterochromatination of a chromosome, and is deeply involved with cell differentiation and individual morphogenesis. Accordingly, the results suggesting that a centromere structure is deeply involved with cell differentiation and individual morphogenesis in some way were obtained by the present invention.

Next, an antibody against Cullin 4 corresponding to the 14^{th} band of Fig. 11 was obtained and HeLa cells were immunostained. The results are shown in Fig. 16 and Fig. 17 with photographs substituted for drawings. Fig. 16A (upper left) shows what was stained with an anti-CENP-A, Fig. 16B (upper right) shows what was stained with an anti-Cullin 4, Fig. 16C (lower left) shows staining of chromosome with DAPI, and Fig. 16D (lower right) is what was obtained by merging the three panels. Fig. 17 shows the results obtained by staining M-phase (left side of each figure) and S-phase (right side of each figure) cells in the same manner as in Fig. 16, respectively, by the same method as in Fig. 16.

As a result, dots were observed in the nuclei, and these dots completely agreed with the dots that CENP-A showed. It is known that Cullin acts as a ubiquitin ligase, and it has a function of eliminating a target protein smoothly via the proteosome pathway by ubiquitinating the target protein. The interesting point of this discovery of the present invention is in that Cullin 4 detected in HeLa cells is identified relatively clearly at S-phase and disappears from M-phase to cell division phase (Fig. 16 and Fig. 17). It is known that an APC complex (Anaphase Promoting Complex) exists when entering chromosome segregation phase from M-phase, and degradation of a certain protein via the ubiquitin pathway becomes a trigger of the onset of mitosis. This discovery is an extremely interesting knowledge which allows us to predict that Cullin 4 plays an important role in such a control of cell cycle. The knowledge that Cullin 4 is localized in a centromere was revealed for the first time by the present invention. The interesting knowledge concerning Cullin 4A is that an increase in Cullin 4A gene or an increase in transcription amount in lung cancer cells is observed. It is known that Cullin 4A binds to DDB1, and it shows the possibility that an increase in the amount of Cullin 4A binding to DDB1 leads to carcinogenesis in some way. The 7^{th} band of Fig. 11 corresponds to DDB 1, and DDB 1 is considered to be a constitutive protein of a centromere. The method of the present invention has been shown to be extremely important to diagnose a cancer or to elucidate the carcinogenesis mechanism.

The present inventors have isolated centromeric chromatin in interphase, not M-phase cells, since the isolated nuclei of HeLa cells are used as the starting material. Therefore, the proteins contained in the isolated centromeric chromatin indicate the function of centromere in interphase nuclei. Also, known proteins other than lots of unknown proteins were contained. Among them, proteins associated with polycomb genes (BMI-1, RNF2, MPC3 or the like) were included. A polycomb gene group is widely involved with differential control that controls gene expression negatively. The fact that this gene group exists in a nucleic centromere in interphase indicates the possibility that a centromeric region also plays an important role in differentiation that decides the future form of a cell. This fact suggests that a centromere responds to a signal when the cell form is decided and it plays a role of a control tower that monitors whole cells or an information center as well as equipartition of a chromosome. This gives a revolutionary aspect as the role of a centromere and is extremely innovative.

As a medical significance of such a function of centromere, it is considered that deficiency of a centromeric function may lead to death in the case of the deficiency of equipartition of a daughter chromosome, however, lead to canceration of cells in the case of the deficiency of a differential control function. Therefore, it is considered that the present invention will open a new way for a cancer research, particularly canceration of cells, and at the same time, it will be useful in diagnosing cancer cells. Accordingly, the present invention provides a method of diagnosing a disease such as a cancer, which is composed of solubilizing centromeric chromatin of a cell and detecting and identifying a protein associated with a disease such as a cancer from proteins obtained by immunoprecipitating the solubilized centromeric chromatin with an anti-CENP antibody.

As the antibody used for immunoprecipitation of the present invention, an anti-CENP antibody can be used, preferably, an anti-CENP-A antibody is used. These antibodies can be used by immobilization on a carrier, for example, a carrier in a form of beads or the like. The precipitated protein according to the method can be analyzed as it is, or can be analyzed after elution treatment with a urea solution or the like. As the analyzing method, it can be analyzed by resolution on SDS-PAGE, a detecting method by an antibody using an antibody against a target protein can be also used, or it can be eluted stepwise with 0.6 M NaCl, 2 M NaCl, pH 2.5, 0.1 M glycine.

The present invention provides an antibody, preferably, a monoclonal antibody against a peptide composed of the N-terminal side of the amino acid sequence of a CENP-A protein, and a method of producing the same. The antibody of the present invention can be also produced by a conventional method, however, preferably, it is produced by the method of producing a monoclonal antibody of the present invention.

The peptide, which is used as an antigen, is characterized in that a peptide composed of a partial-length amino acid sequence of a CENP-A protein is used. Since 2/3 of the C-terminal side of the CENP-A protein is composed of an amino acid sequence that is homologous to an H3 protein forming a histone core, it is preferable to use a sequence unique to the CENP-A protein composed of about 1/3 of the N-terminal side of the CENP-A protein. The preferable amino acid sequence includes a peptide containing the amino acid sequence of 3- to 19-positions of the CENP-A protein, a peptide containing the amino acid sequence of 27- to 40-positions thereof and a peptide containing the amino acid sequence of 126- to 140-positions thereof. More preferable peptide includes a peptide containing the amino acid sequence of 3- to 19-positions of the CENP-A protein, further preferably, a peptide composed of the amino acid sequence of 3- to 19-positions thereof.

A method of producing an antibody of the present invention uses a method of producing a monoclonal antibody in a common cell fusion method, and is characterized in that P3-X63-Ag8.653 cell, which is a mouse myeloma cell, is used as a myeloma cell, and GIT medium is used as a medium. The GIT medium is available from Nihon Pharmaceutical Co., Ltd., however, it is not limited thereto, and any medium as long as it is the same kind and does not require fetal bovine serum can be used.

As the antigen for the method of the present invention, various antigens, which can produce an antibody by a common method, can be used. For example, a recombinant protein of a CENP-B protein or its N-terminal peptide or the like using an E. coli expression system, a recombinant protein of a CENP-C protein or the like using a baculovirus system, other proteins or a fragment thereof can be used.

### Examples

The present invention will be illustrated in more detail with reference to Examples, however, the present invention is not limited to these Examples.

### Example 1 (Production of monoclonal antibodies against peptides on N-terminal side of CENP-A protein)

Human CENP-A peptides with various amino acid lengths (amino acids 3-19 from the amino-terminus) were chemically synthesized and antibodies were produced using various fragments of a CENP-A protein. More specifically, a fragment (#3-19) composed of the amino acid sequence of 3- to 19-positions of the human CENP-A protein, a fragment (#12-25) composed of the amino acid sequence of 12- to 25-positions of the human CENP-A protein, a fragment (#27-40) composed of the amino acid sequence of 27- to 40-positions of the human CENP-A protein, a fragment (#39-53) composed of the amino acid sequence of 39- to 53-positions of the human CENP-A protein and a fragment (#126-110) composed of the amino acid sequence of 126- to 140-positions of the human CENP-A protein are used as respective antigens.

Each peptide was covalently linked to keyhole limpet haemocyanin (Pierce). By using mouse myeloma cells (P3-X63-Ag8.653), fused cells were cultured with GIT medium (Nihon Pharmaceutical Co., Ltd.). The specific procedures such as a fusing method and a screening method were conducted according to the methods of Coligan et al. (Coligan J.E., et al., "Current protocols in immunology", Greene Publishing Associates and Wiley-Interscience).

By Western blot analysis, the selected hybridoma cells produced IgG that specifically recognized the CENP-A protein, and centromere regions in S-phase and M-phase were labeled by indirect immunofluorescence microscopy (see Fig. 3A).

As a result, it became clear that the obtained anti-CENP-A peptide antibodies (#3-19, #27-40 and #126-140) were extremely useful antibodies. In particular, #3-19 antibody gave the excellent results in the Western blotting method, the indirect immunofluorescence staining and the immunoprecipitation method.

### Example 2 (Production of monoclonal antibodies against peptides on N-terminal side of CENP-B protein)

Antibodies were produced in the same method as in Example 1 using, as an antigen, an amino-terminal portion of CENP-B which had been isolated as a clathrate expressed in E. coli cells.

### Example 3 (Production of anti-CENP-C antibodies)

The DNA sequence that codes for the half amino-terminal of human CENP-C (amino acids 1-426) was amplified by PCR using a cDNA library of HeLa cells, and cloned into pFastBac HT vector (Gibco-BRL) after digestion with NcoI and EcoRI. The primers used were 5'-GGCCGGACCATGGCTGCGTCCGGTCTGGA and 5'-ATAGAATCCTCTTCAGCTGGTTTAGCCAT. The isolated clones were sequenced, and a clone without amino acid substitution was selected. The half amino-terminal of CENP-C was expressed in SF9 cells using the Bac-to-Bac baculovirus expression system (Gibco-BRL) and purified on an Ni-column. The polyclonal guinea pig antibodies were affinity purified with the same protein coupled to CN-Br-activated Sepharose 4B beads (Amersham Pharmacia Biotech).

### Example 4 (Solubilization of chromatin)

HeLa S3 cells were cultured with RPMI1640 medium (Nissui Pharmaceutical, Tokyo, Japan) containing 5% bovine serum until the concentration reached 5×10⁵ cells/ml. The nuclei of the cultured cells were separated.

The nuclear pellet (1 × 10⁹ nuclei) of HeLa cells was suspended in 5 ml of ice-cold WB (WB: 20 mM Hepes pH 8.0, 20 mM KCI, 0.5 mM EDTA, 0.5 mM dithiothreitol (DDT), 0.05 mM phenylmethylsulfonyl fluoride (PMSF)) containing 0.3 M NaCl. The nuclear suspension was digested with micrococcal nuclease (MNase) at 37°C after the addition of CaCl₂ to a final concentration of 2 mM. The reaction was stopped by the addition of EGTA to a final concentration of 10 mM with rapid cooling. The digests were centrifuged using an R20A2 rotor (Hitachi) at 10,000 g for 10 minutes at 4°C. The solubilized chromatin was recovered in the supernatant. The pellet was resuspended in 5 ml of the same buffer.

The results are shown in Fig. 1. Fig. 1A shows the results of examining the conditions for solubilizing centromeric chromatin from the nuclei of HeLa cells. Fig. 1B shows the size distribution of the DNA in the chromatin after MNase digestion. Fig. 1C shows the results of detecting a soluble fraction and an insoluble fraction by staining with Coomassie brilliant blue (CBB) after digesting bulk chromatin proteins with MNase and fractionating a soluble fraction and an insoluble fraction by SDS-PAGE (Fig. 1C, upper panel), and the results of identifying CENP-A by immunostaining with the use of an ACA antibody after transferring the fractionated proteins to a membrane (Fig. 1C. lower panel).

### Example 5 (Immunoprecipitation)

ACA serum (MI) includes IgG antibodies that recognize CENP-A, CENP-B and CENP-C. These IgGs were purified by adsorbing them onto protein G-Sepharose columns (Pharmacia). Guinea pig anti-CENP-C IgG (gpαCC) was affinity purified on a CENP-C-conjugated Sepharose 4B column. Each of these IgGs (about 10 mg each) was conjugated to CNBr-activated Sepharose 4B (1 g of dry powder; Pharmacia).

These IgG-conjugated Sepharose beads (50 µl) were added to the solubilized chromatin samples (1 ml), produced in Example 4, supplemented with 0.1 % NP-40, or to respective fractions from glycerol gradient sediment (2 ml) and incubated at 4°C for 6 hours with rotation. For CHIP with anti-CENP-B antibodies, anti-CENP-B IgG and protein G-Sepharose were added to the sample and incubated at 4°C for 7 hours with rotation. After the incubation, the beads were washed five times with 0.3 M NaCl -WB containing 0.1 % Tween-20 at room temperature for 3 minutes, and resuspended in 50 to 100 µl of SDS buffer (50 mM Tris-HCl pH 8.0,25 mM DTT, 1% SDS, 15% glycerol). The obtained proteins were analyzed by SDS-PAGE and Western blotting.

### Example 6 (SDS-PAGE and Western blotting)

The proteins obtained by the method of Example 5 were separated by 12.5% SDS-PAGE and transferred to a polyvinylidene difluoride membrane (PVDF) (Millipore) by a known method. The membrane was pretreated with 10% skimmed milk (Wako), reacted with ACA serum (A.K., 1:3,000 dilution) overnight at 4°C and reacted with goat anti-human immunoglobulin G (IgG) conjugated to horseradish peroxidase (1:3,000 dilution) (Bio-Rad) overnight at 4°C. Color development was carried out with Konica immunostain (Konica). To measure the intensity of the protein bands stained with Coomassie brilliant blue or horseradish peroxidase, an image was digitalized by a scanner and analyzed using the NIH image 1.55.

The results are shown in Fig. 4. Fig. 4 shows the results of CENP-A, -B, and -C by Western blotting, using the ACA serum, of the samples (αCA) precipitated with the anti-CENP-A antibody of the present invention, and the samples (ACA) obtained by further precipitating the supernatants with the ACA antibody IgG. Fig. 5 is a graph showing the results obtained by calculating the molar ratios of CENP-C/B in the precipitated A/B/C complex in Fig. 4. Fig. 6 is a graph showing the molar ratios of CENP-A/B and the amounts of CENP-A and CENP-B in each sample of the results of Fig. 4.

### Example 7 (glycerol gradient sedimentation)

A portion of 3 ml of the solubilized chromatin solution obtained in Example 4 was added to 34 ml of 5 to 20% glycerol containing 50 mM Tris-HCl (pH 8.0), 2 mM EDTA, 0.1% NP-40, 2 mM DTT, and 0.15 M NaCl layered onto 1 ml of 50% glycerol and centrifuged using an SW28 rotor (Beckman) at 22,000 rpm for 15.5 hours at 4°C. Then 2 ml of aliquots were fractionated from the bottom, and DNA and proteins were separated by a 1 % agarose gel electrophoresis and SDA-PAGE, respectively.

The results are shown in Fig. 7. Fig. 7A shows the distribution of the bulk chromatin of sample #3 of Fig. 1B by nucleosome DNA ladders. Figs. 7B and 7C show what was obtained by detecting distributions of CENP-A, CENP-B, and CENP-C by the Western blotting method using ACA serum after the immunoprecipitation of each fraction with an anti-CENP-A antibody (Fig. 7B) or an anti-CENP-C antibody (Fig. 7C).

### Example 8 (Cloning and sequencing of the DNA recovered by CHIP using anti-CENP-A, -B, or -C antibody)

HeLa nuclei (2 x 10⁸ nuclei/ml, 15 ml) were digested with 40 U/ml of MNase (Boehringer) at 37°C for 10 minutes. To remove short DNA fragments and free proteins, the soluble fraction of the digest was centrifuged through a 5 to 20% glycerol gradient, and a fraction heavier than trinucleosome was pooled and immunoprecipitated using an anti-CENP-A, -B, or -C antibody. After the immunoprecipitation, the beads were treated with proteinase K, and the DNA was subsequently purified by phenol extraction. Gel mobility shift competition assay using the precipitated DNA and the purified CENP-B was performed. The DNA was cloned into 3'dT-terminal vector pCR2.1-TOPO using the TOPO TA cloning kit (Invitrogen) after the treatment with Taq polymerase and complementary 3' A terminus was added. The DNA sequences were determined using an ABI PRISM 3700 DNA analyzer (Perkin Elmer Applied Biosystems) or an ABI PRISM 377 DNA sequencer (Perkin Elmer Applied Biosystems). The sequences were compared with the database using the BLAST server at NCBI. Clones showing homology to α-satellite DNA were classified according to the classification of Alexandrov et al.

### Example 9 (Production of the A/B/C control mixture)

CENP-A, CENP-B, and CENP-C were expressed using the baculovirus Bac-to-Bac system (Gibco-BRL) and purified as follows. CENP-A and histone H4 were coexpressed with 6 histidines at the amino terminus, and the CENP-A/his6-H4 complex was purified using an Ni-column. CENP-B was purified on a Q-Sepharose column. The CENP-C gene encoding the full length CENP-C was amplified by PCR using a cDNA library of HeLa cells (Clontech), and ligated to pFastBac vector. The full length CENP-C was expressed in the baculovirus system and isolated as a clathrate. It was solubilized with 6 M urea and purified on a heparin-Sepharose column. The concentration of each protein was calculated from the protein band intensity after CBB staining of SDS-PAGE, using bovine serum albumin (ESA) as a standard. The A/B/C control mixture contains 61 finol/ml of CENP-A, 23 fmol/ml of CENP-B, and 25 fmol/ml of CENP-C, and A/B/C ratio is 2.65:1:1.09. To prevent loss during handling of the proteins, BSA and core histones were added at the concentrations of 75 ng/ml and 37 ng/ml, respectively. The reactivity of the recombinant proteins and ACA serum was confirmed to be equivalent by directly compared with the results of the Western blotting using CENP-A, -B and -C purified from HeLa cells.

### Example 10

According to the method described in Example 4, the isolated nuclei of HeLa cells were digested with MNase (6 U/ml, 37°C, 30 minutes), and solubilized. From the obtained bulk chromatin, centromeric chromatin was isolated using the anti-CENP-A antibody beads according to the method described in Example 5.

The results of analyzing DNA contained in the isolated centromeric chromatin are shown in Fig. 8.

Next, proteins contained in the isolated centromeric chromatin was digested with proteinase K, and DNA was extracted by phenol treatment. The DNA was radiolabeled with γ³²P-ATP using polynucleotide kinase, and loaded on a 1% agarose gel.

The isolated centromeric chromatin contained CENP-H, CENP-I/H. Mis-6, H. Mis-12. The results are shown in Fig. 9.

### Example 11

The solubilized centromeric chromatin obtained in Example 10 was immunoprecipitated with an α-CENP-A antibody according to the method described in Example 5.

Fig. 10 is a photograph substituted for a drawing which shows the results of the proteome analysis of the substances precipitated by immunoprecipitation (CHIP), using an α-CENP-A antibody, of the centromeric chromatin (the same samples as in Fig. 8 and Fig.9) which had been solubilized by MNase treatment.

Lanes 2, 3 and 4 show the numbers of fractions obtained by eluting the precipitated substances with 8 M urea. This is silver-staining on 10% SDS-PAGE. Lane M corresponds to a molecular weight marker. The right end of the SDS was what was boiled. The numbers on the right end show the number of bands obtained by conducting proteome analysis using the respective SDS-boil samples. The descriptions of the proteins of respective bands are described in context.

Fig. 11 is a photograph substituted for a drawing which shows the results of the Coomassie staining after separating, by 7.5% SDS-PAGE, proteins of lane 3 sample eluted with 8 M urea in Fig. 10.

### Example 12 (Non-specific adsorption by IgG-conjugated beads)

Nuclei were isolated and purified from HeLa cells (5 × 10⁹) and bulk chromatin was solubilized by digestion with MNase in the presence of 0.3 M NaCl. IgG-Sepharose beads were added thereto and incubated overnight at 4°C with mixing by rotation so that adsorption proceeded. After the reaction, the supernatant was mixed by the anti-CENP-A antibody-sepharose beads in the same way and left overnight and the beads were recovered. The respective beads were washed three times with a buffer containing 0.3 M NaCl and proteins were eluted stepwise with 0.6 M NaCl, 2 M NaCl, 0.1 M glycine-HCl (pH 2.5). The samples obtained by elution of the respective beads with 0.6 M NaCl were separated by 12.5% SDS-PAGE and stained with Coomassie brilliant blue (CBB).

The results are shown in Fig. 12. In Fig. 12, lane IgG on the left; IgG beads, lane α-CENP-A in the center; anti-CENP-A antibody beads, lane M on the right; molecular weight marker. The numbers on the left side of the figure show the major bands in IgG lane and the figure show the analysis results by proteome analysis of these bands. In fact, all the gels were cut out and analyzed exhaustively. The results of as many as 200 amino acid sequences were obtained, however, only major proteins are shown here.

Further, samples (αA and Ig) eluted with 0.6 M NaCl from the obtained respective beads were separated by 12.5% SDS-PAGE, transferred to a PVDF membrane, and immunostained with the antibodies shown in the upper part of the figure.

The results are shown in Fig. 13. The left of each lane set of Fig. 13 corresponds to the αCA beads sample and the right corresponds to the IgG beads sample. The primary antibodies were a rabbit anti-CENP-H antibody in the lane set 1, a rabbit anti-CENP-H antibody and an ACA antibody in the lane set 2, a mouse anti-mouse Ring1B antibody (monoclonal antibody) in the lane set 3, a rabbit anti-human RNF2 antibody in the lane set 4, a mouse anti-human BMI-1 antibody in the lane set 5, a rabbit anti-CENP-I/H. Mis6 antibody in the lane set 6 and a rabbit anti-H. Mis12 antibody in the lane set 7. As the secondary antibodies, an anti-human IgG antibody (lane 2), an anti-mouse IgG antibody (lanes 3 and 5) and an anti-rabbit IgG antibody conjugated to horseradish peroxidase were used. Color development was carried out with Konica immunostain.

### Industrial Applicability

The present invention provides an antibody that reacts highly sensitively and surely with a CENP-A protein. The antibody of the present invention has a high specific affinity for a centromeric nucleosome, therefore the constitutive factors of the structure of the centromeric region (kinetochore) can be exhaustively isolated. For example, the antibody (#3-19) of the present invention, recognized the target CENP-A protein surely and highly sensitively by chromatin immunoprecipitation (CHIP) analysis following the solubilization of the chromatin of HeLa cells using micrococcal nuclease (MNase). As a result, it became clear that CENP-A, CENP-B and CENP-C form a complex to form a basic structure of the kinetochore region. It is expected that the other antibodies will be effective for the same purpose. By using these antibodies in combination, centromeric chromatin can be purified with much higher purity, therefore, it is expected that it will be usable for isolation of a centromere/kinetochore complex, exhaustive analysis of constitutive factors and gene identification (proteomics) in the future.

Also, according to the method of producing an antibody of the present invention, a monoclonal antibody can be easily and simply screened from a large number of antigens.

Also, the present invention provides a method of solubilizing chromatin. The solubilization of the present invention fractionates chromatin, however, it is characterized in that fragmentation and solubilization are conducted while overall characteristics of chromatin are retained. In addition, the solubilization method of the present invention is simple and excellent in reproducibility, and has a beneficial effect on an analysis of chromatin proteins. Therefore, the present invention provides an immunoprecipitation method of chromatin solubilized by the method of the present invention, and an analysis method of chromatin proteins by the immunoprecipitation method. By analyzing chromatin proteins, it is possible to analyze the state of cell differentiation, cell transmutation, cell canceration or the like.

## Claims

1. An antibody, which is against a partial-length peptide of a CENP-A protein

2. The antibody according to claim 1, which is an antibody against a peptide containing a part of the 1/3 of the N-terminal portion of the amino acid sequence of the CENP-A protein.

3. The antibody according to claim 1 or 2, wherein the partial-length peptide of the CENP-A protein is a peptide containing the amino acid sequence of 3- to 19-positions of the CENP-A protein, a peptide containing the amino acid sequence of 27- to 40-positions thereof or a peptide containing the amino acid sequence of 126- to 140-positions thereof.

4. The antibody according to claim 3, wherein the partial-length peptide of the CENP-A protein is the peptide containing the amino acid sequence of 3- to 19-positions of the CENP-A protein.

5. The antibody according to any one of claims 1 to 4, wherein the CENP-A protein is a human CENP-A protein.

6. The antibody according to any one of claims 1 to 5, wherein the antibody is a monoclonal antibody.

7. A method of producing a monoclonal antibody against a CENP-A protein **characterized in that**, in a method of producing a monoclonal antibody by a cell fusion method, the CENP-A protein is used as an antigen, P3-X63-Ag8.653 cell, which is a mouse myeloma cell, is used as a myeloma cell, and GIT medium is used as a medium.

8. The method according to claim 7, wherein the N-terminal portion of the CENP-A protein is used as an antigen.

9. A method of solubilizing centromeric chromatin using MNase.

10. The method according to claim 9, wherein a concentration of the MNase is 1 U/ml to 500 U/ml and a treating time is 1 to 100 minutes.

11. A method of solubilizing centromeric chromatin using MNase and immunoprecipitating the obtained solubilized substance using an anti-CENP antibody.

12. The method according to claim 11, wherein the anti-CENP antibody is an antibody immobilized on a carrier.

13. The method according to claim 11 or 12, wherein the anti-CENP antibody is an anti-CENP-A antibody.

14. A method of solubilizing centromeric chromatin using MNase, immunoprecipitating the obtained solubilized substance using an anti-CENP antibody and detecting and identifying the precipitated protein.

15. The method according to claim 11 or 14, wherein a protein to be detected and identified is a protein associated with differentiation.
